# FASCICULE DE BREVET EUROPEEN

(11) **EP 0 565 396 B1**
(45) Date de publication et mention de la délivrance du brevet: **07.01.1998**
(21) Numéro de dépôt: 93400772.5
(22) Date de dépôt: 25.03.1993
(51) Int. Cl.: C07D 401/06, C07D 401/12, C07D 401/14, A61K 31/415

(54) **Dérivés de 1-[2-(arylsulfonylamino)-1-oxoéthyl]pipéridine, leur préparation et leur application en thérapeutique**
1-[2-(Arylsulfonylamino)-1-oxoethyl]piperidin-Derivate, ihre Herstellung und therapeutische Verwendung
1-[2-(arylsulfonylamino)-1-oxoethyl]piperidine derivatives, their preparation and use in therapy

(30) Priorité: 30.03.1992 FR 9203828
(43) Date de publication de la demande: 13.10.1993
(62) Demande divisionnaire de: 93114975.1
(73) Titulaire: SYNTHELABO, 92350 Le Plessis Robinson (FR)
(72) Inventeur: Lassalle, Gilbert, F-92140 Clamart (FR); Purcell, Thomas, F-78490 Montfort l'Amaury (FR); Galtier, Daniel, F-78280 Guyancourt (FR); Williams, Paul Howard, F-75011 Paris (FR); Galli, Frédéric, F-78170 La Celle St Cloud (FR)
(74) Mandataire: Thouret-Lemaitre, Elisabeth

(56) Documents cités:
- EP-A- 0 008 746
- EP-A- 0 097 630
- CARDIOVASC. DRUG REV. (CDREEA); 91; VOL.9 (3); PP.247-63 'Argatroban, a selective, potent thrombin inhibitor'
- CHEMICAL ABSTRACTS, vol. 111 Columbus, Ohio, US; abstract no. 58337, ISHIKAWA F '.alpha.-(2-Imino-1,2,3,4,5,6-h exahydro-4-pyrimidinyl)-N.alpha.-aryl sulfonylglycine derivatives as antithrombotics'

## Description

La présente invention a pour objet des dérivés de 1-[2-(arylsulfonylamino)-1-oxoéthyl]pipéridine, leur préparation et leur application en thérapeutique.

Les composés de l'invention répondent à la formule (I) 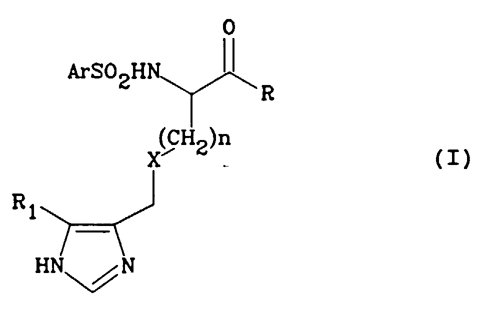 dans laquelle
R représente un groupe 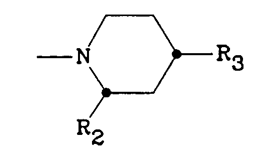 où R₂ est un atome d'hydrogène, un groupe (C₁-C₄)alcoxycarbonyle, un groupe carboxylique, un groupe carboxylate de sodium, un groupe -CH₂OR₄, R₄ étant un atome d'hydrogène, un groupe (C₁-C₄)alkyle ou un groupe (C₁-C₄)acyle, un groupe amide de formule -CONR₅R₆ ou un groupe -CN₄R₅, R₅ étant un atome d'hydrogène ou un groupe (C₁-C₄)alkyle et R₆ étant un atome d'hydrogène, un groupe (C₁-C₄)alkyle, un groupe hydroxy, un groupe (C₁-C₄)alcoxy ou un groupe (C₁-C₃)alcoxyphényle et R₃ est un groupe (C₁-C₄)alkyle,
R₁ représente soit un atome d'hydrogène, soit un groupe (C₁-C₄)alkyle,
X représente soit un atome de soufre, soit un atome d'oxygène, soit un groupe méthylène,
n = 1 ou 2 et
Ar représente soit un groupe naphtalén-1-yle substitué par un groupe di(C₁-C₄)alkylamino, soit un groupe 6,7-di(C₁-C₄)alcoxy naphtalén-1-yle, soit un groupe quinoléin-8-yle substitué en 3 par un groupe (C₁-C₄)alkyle, soit un groupe 1,2,3,4-tétrahydroquinoléin-8-yle substitué en 3 par un groupe (C₁-C₄)alkyle, soit un groupe 1*H*-indazol-7-yle.

Les composés préférés de l'invention sont ceux répondant à la formule (I) dans laquelle,
R représente un groupe 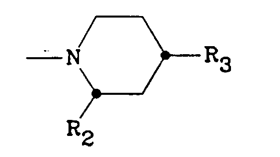 où R₂ est un atome d'hydrogène, un groupe (C₁-C₄)alcoxycarbonyle, un groupe carboxylique, un groupe carboxylate de sodium, un groupe -CH₂OR₄, R₄ étant un atome d'hydrogène, un groupe (C₁-C₄)alkyle ou un groupe (C₁-C₄)acyle, un groupe amide de formule -CONR₅R₆ ou un groupe -CN₄R₅, R₅ étant un atome d'hydrogène ou un groupe (C₁-C₄)alkyle et R₆ étant un atome d'hydrogène, un groupe (C₁-C₄)alkyle, un groupe hydroxy, un groupe (C₁-C₄)alcoxy ou un groupe (C₁-C₃)alcoxyphényle et R₃ est un groupe (C₁-C₄)alkyle,
R₁ représente soit un atome d'hydrogène, soit un groupe (C₁-C₄)alkyle,
X représente soit un atome de soufre, soit un atome d'oxygène, soit un groupe méthylène,
n = 1 ou 2 et
Ar représente soit le groupe 5-(diméthylamino)-naphtalén-1-yle, soit le groupe 6,7-diméthoxy-naphtalén-1-yle, soit le groupe 3-méthyl-quinoléin-8-yle, soit le groupe 3-méthyl-1,2,3,4-tétrahydroquinoléin-8-yle.

Parmi ces composés, les composés de choix de l'invention sont ceux répondant à la formule (I) dans laquelle
R représente un groupe 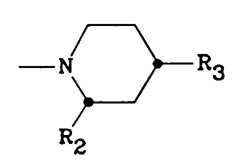 où R₂ est un groupe carboxylique, un groupe éthoxycarbonyle, un groupe hydroxycarboxamide, un groupe hydroxyméthyle, ou un groupe 1*H*-tétrazolyle et R₃ est un groupe méthyle ou un groupe éthyle,
R₁ est un atome d'hydrogène ou un groupe méthyle,
X est un groupe méthylène
n = 1 et
Ar est le radical 3-méthyl-1,2,3,4-tétrahydroquinoléin-8-yle.

Les composés de l'invention possèdent 3 centres asymétriques selon la signification des substituants.
La configuration préférentielle du groupe pipéridinyle est [2R,4R].
La configuration préférentielle du carbone asymétrique de la partie acide aminé centrale 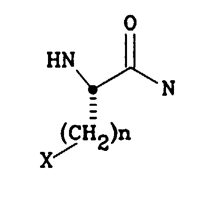 est [S].
De plus il existe un quatrième centre asymétrique lorsque Ar représente un groupe 1,2,3,4-tétrahydroquinoléin-8-yle substitué en 3 par un groupe (C₁-C₄)alkyle. Dans ce cas les composés existent sous forme d'un mélange de diastéréoisomères.

Les composés de l'invention peuvent exister sous forme de bases libres ou de sels d'addition aux acides pharmaceutiquement acceptables.

Dans les schémas de synthèse 1 à 4 qui suivent, R₁, R₂, R₃ et Ar sont tels que définis précédemment et Ts représente un groupe 4-(méthylphényl)-sulfonyle.

Les composés de l'invention, pour lesquels X représente un groupe méthylène, répondent à la formule (I bis) 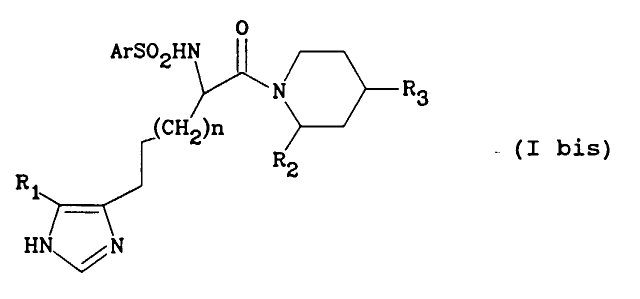 dans laquelle n = 1 ou 2 et R₁, R₂, R₃ et Ar sont tels que définis précédemment; ils peuvent être synthétisés selon le schéma 1 de la page suivante.
Dans une première étape, on fait réagir un alcool de formule (II) avec du chlorure de thionyle dans un mélange de diméthylformamide/dichlorométhane puis on fait réagir le composé obtenu avec de la triphénylphosphine dans un solvant tel que le diméthylformamide ou le benzène à une température de 80°C; on obtient un chlorure de triphénylméthyl phosphonium de formule (III).
Dans une deuxième étape, on fait réagir le composé (III) avec un composé de formule (IV); la réaction est conduite dans un solvant tel que le tétrahydrofurane, en présence de n-butyllithium à une température de - 70°C. On obtient un composé de formule (V), sous forme d'un mélange cis/trans au niveau de la double liaison.
Dans une troisième étape, on soumet le composé (V) obtenu précédemment à une hydrogénation catalytique pour obtenir un composé de formule (VI).
Dans une quatrième étape, on fait réagir ce composé avec un chlorure d'arylsulfonyle de formule ArSO₂Cl dans laquelle Ar est tel que défini ci-dessus, et on obtient un composé de formule (VII); la réaction est conduite dans un solvant tel que le chloroforme ou le dichlorométhane en présence d'une base comme la triéthylamine.

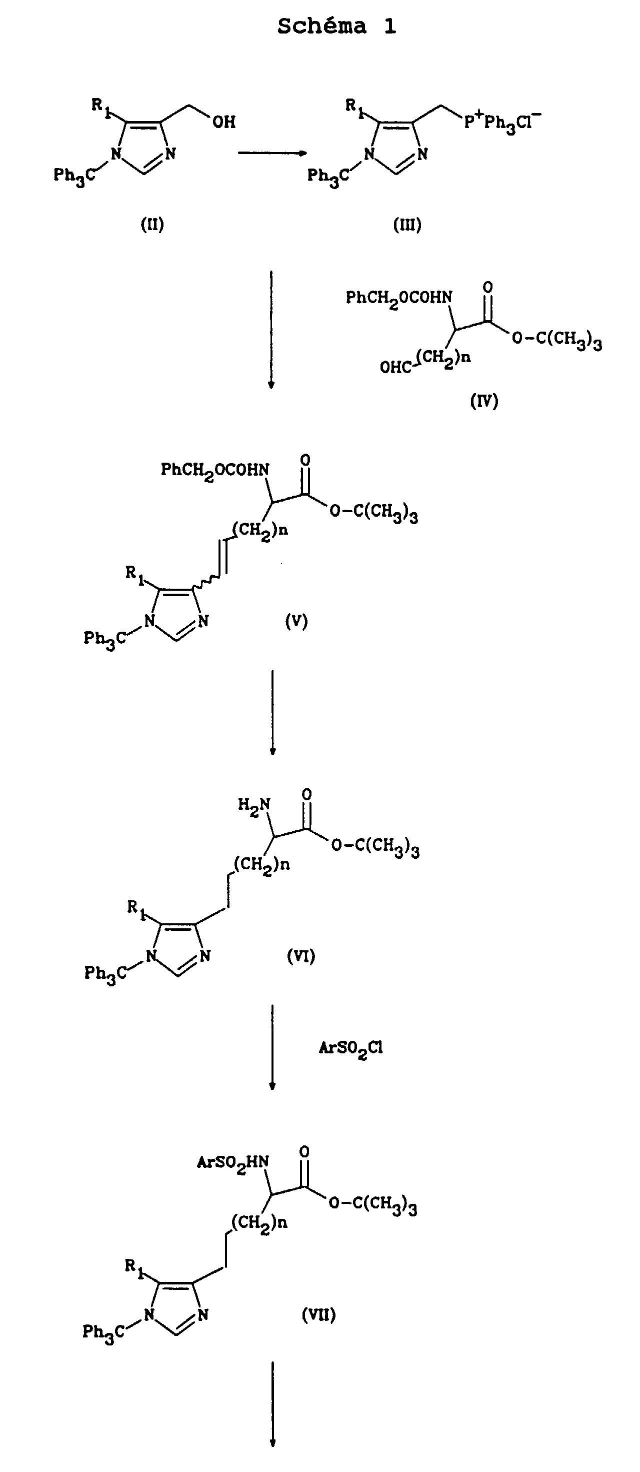

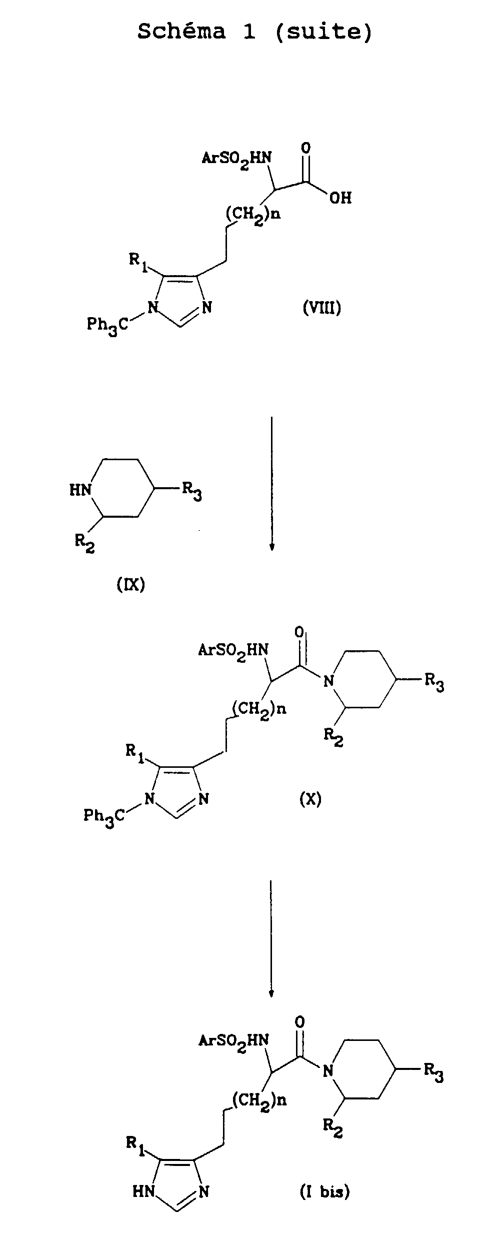

Dans une cinquième étape, on réalise la déprotection du composé (VII) en milieu acide chlorhydrique gazeux dans un solvant tel que le benzène pour obtenir un composé de formule (VIII).
Dans une sixième étape, on réalise une condensation du composé (VIII) avec un composé de formule (IX) et on obtient un composé de formule (X). La réaction est réalisée en présence d'une base comme la 4-méthylmorpholine ou la N,N-diisopropyléthylamine (base de "Hunig") et d'un agent couplant tel que l'hexafluorophosphate de [(benzotriazol-1-yl)-oxy] tris(diméthylamino)phosphonium, le diphénylphosphorylazide ou le chlorhydrate de 1-(3-diméthylaminopropyl)-3-éthylcarbodiimide couplé avec le 1-hydroxybenzotriazole.
Dans une septième étape, on réalise la déprotection du noyau imidazole du composé (X) soit par hydrogénation, soit par action de l'acide acétique dans un mélange tétrahydrofurane/eau. On obtient un composé de formule (I bis). Lorsqu'on hydrogène, avec chauffage, un composé de formule (X) dans laquelle Ar représente le groupe 3-méthyl-quinoléin-8-yle, on obtient des composés de formule (I bis) dans laquelle Ar est le groupe 3-méthyl-1,2,3,4-tétrahydroquinoléin-8-yle. Lorsque R₂ est un groupe (C₁-C₄)alcoxycarbonyle, on peut faire réagir le composé (I bis) avec une solution aqueuse 1 N d'hydroxyde de sodium pour obtenir le sel de l'acide correspondant.

Les composés de l'invention, pour lesquels X représente un atome de soufre, répondent à la formule (I ter) 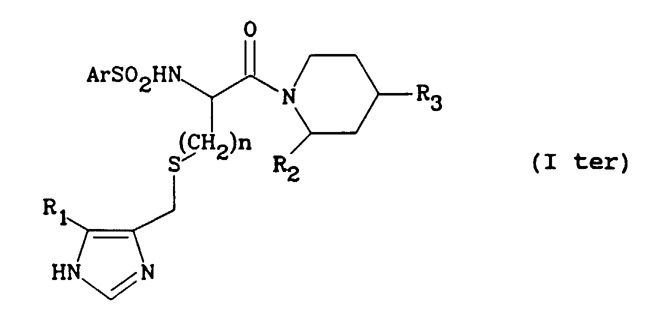 dans laquelle n = 1 ou 2 et R₁, R₂, R₃ et Ar sont tels que définis précédemment.
Les composés répondant à la formule (I ter) dans laquelle n = 1 peuvent être synthétisés selon le schéma 2 de la page suivante.

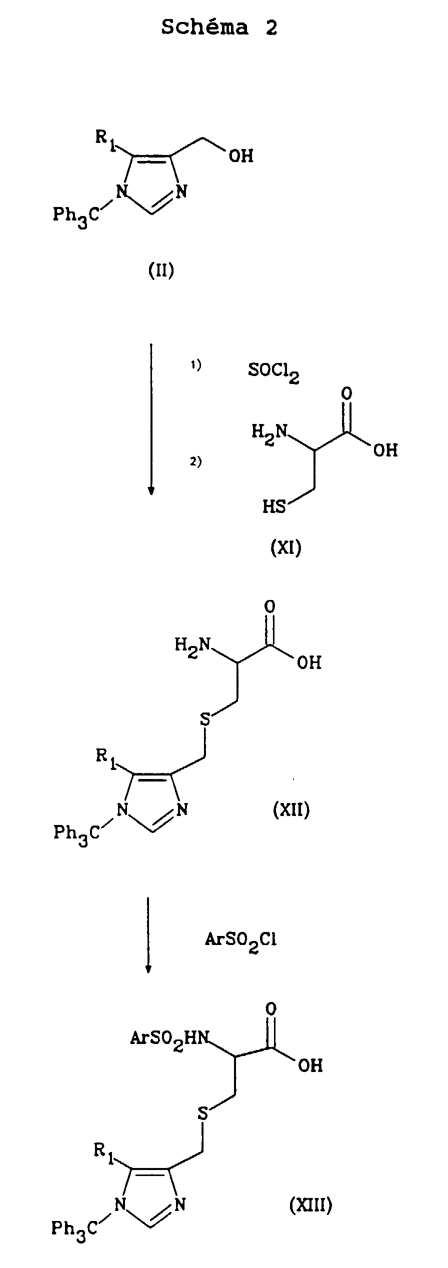

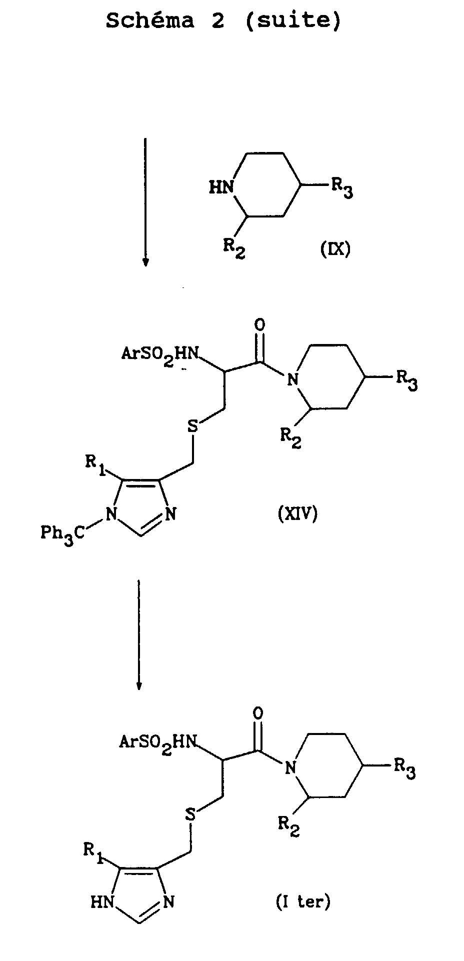

Dans une première étape, on fait réagir un alcool de formule (II) avec du chlorure de thionyle dans un mélange diméthylformamide/dichlorométhane puis on fait réagir le composé obtenu avec de la *L*-cystéine (XI) et on obtient un composé de formule (XII); la réaction est conduite dans une solution aqueuse 1 N d'hydroxyde de sodium.
Dans une deuxième étape, on fait réagir le composé de formule (XII) avec un chlorure d'arylsulfonyle de formule ArSO₂Cl dans laquelle Ar est tel que défini ci-dessus, et on obtient un composé de formule (XIII); la réaction est conduite dans une solution aqueuse d'hydroxyde de sodium.
Dans une troisième étape, on réalise une condensation avec un composé de formule (IX) et on obtient un composé de formule (XIV). La réaction est réalisée en présence d'une base comme la 4-méthylmorpholine ou la N,N-diisopropyléthylamine (base de "Hunig") et d'un agent couplant tel que l'hexafluorophosphate de [(benzotriazol-1-yl)oxy]tris(diméthylamino) phosphonium, le diphénylphosphorylazide ou le chlorhydrate de 1-(3-diméthyl-aminopropyl)-3-éthylcarbodiimide couplé avec le 1-hydroxy-benzotriazole.
Dans une quatrième étape, on réalise la déprotection du noyau imidazolé du composé (XIV) soit par hydrogénation, soit par action de l'acide acétique dans un mélange tétrahydrofurane/eau. On obtient un composé de formule (I ter). Lorsqu'on hydrogène, avec chauffage, un composé de formule (XIV) dans laquelle Ar représente le groupe 3-méthyl-quinoléin-8-yle, on obtient des composés de formule (I ter) dans laquelle Ar est le groupe 3-méthyl-1,2,3,4-tétrahydro-quinoléin-8-yle.
Lorsque R₂ est un groupe (C₁-C₄)alcoxycarbonyle, on peut faire réagir le composé (I ter) avec une solution aqueuse d'hydroxyde de sodium pour obtenir le sel de l'acide correspondant.

Les composés répondant à la formule (I ter) dans laquelle n = 2 peuvent être synthétisés selon le schéma 3 de la page suivante.
Dans une première étape, on fait réagir un alcool de formule (II) avec du chlorure de thionyle dans un mélange diméthylformamide/dichlorométhane puis on fait réagir le composé obtenu avec du thioacétate de potassium. On obtient un composé de formule (XV).
Dans une deuxième étape, on fait réagir le composé (XV) avec un tosylate de formule (XVI) pour obtenir un composé de formule (XVII); cette réaction est conduite en présence d'une base comme le méthylate de sodium. Le tosylate de formule (XVI) est obtenu par action du chlorure de paratoluènesulfonyle, en présence d'une base, sur l'alcool précurseur du composé de formule (IV) dont la synthèse est décrite par Valerio R.M. et coll. dans Synthesis, 1988, 786.

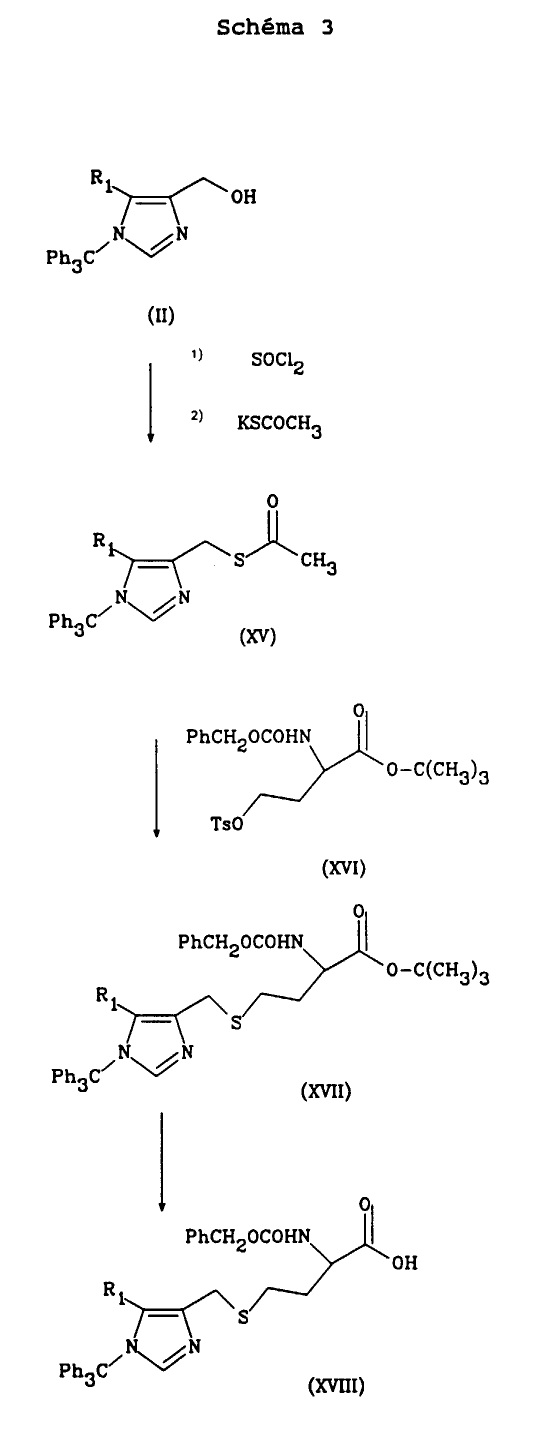

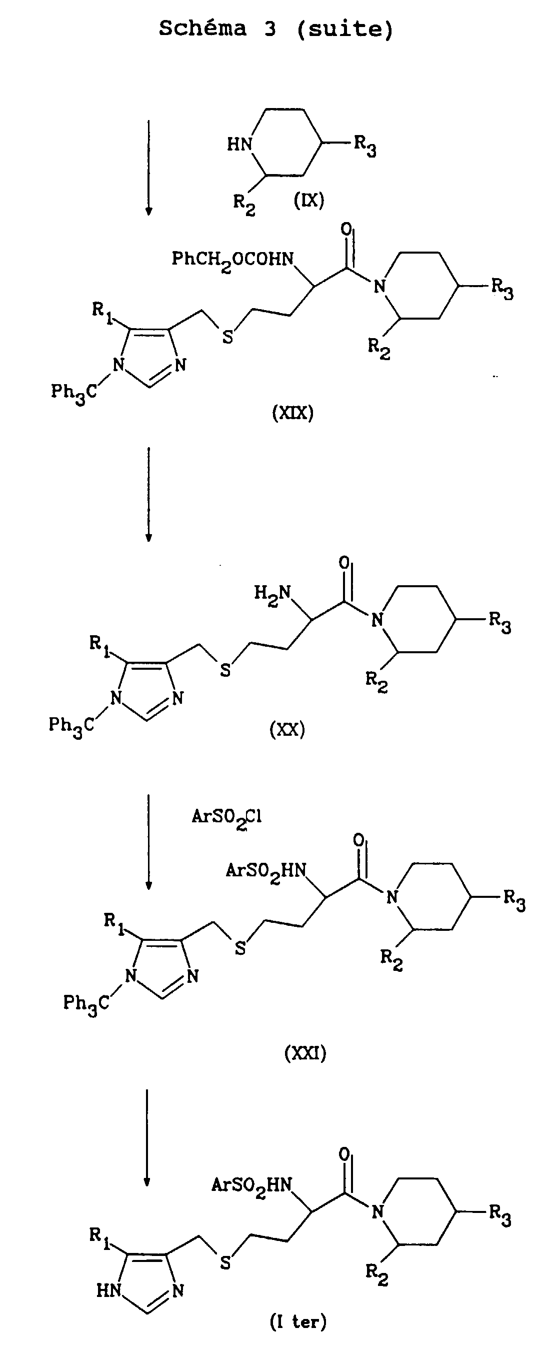

Dans une troisième étape, on déprotège le composé de formule (XVII) dans l'acide chlorhydrique gazeux dans le benzène et on obtient un composé de formule (XVIII).
Dans une quatrième étape, on réalise la condensation d'un composé de formule (XVIII) avec un composé de formule (IX) et on obtient un composé de formule (XIX). La réaction est réalisée en présence d'une base comme la 4-méthylmorpholine ou la N,N-diisopropyléthylamine (base de "Hunig") et d'un agent couplant tel que l'hexafluorophosphate de [(benzotriazol-1-yl)-oxy]tris(diméthylamino)phos-phonium, le diphénylphosphorylazide ou le chlorhydrate de 1-(3-diméthyl-aminopropyl)-3-éthylcarbodiimide couplé avec le 1-hydroxybenzotriazole.
Dans une cinquième étape, on déprotège la fonction amine du composé (XIX), de préférence en milieu acide, pour obtenir un composé de formule (XX).
Dans une sixième étape, on fait réagir le composé de formule (XX) avec un chlorure d'arylsulfonyle de formule ArSO₂Cl dans laquelle Ar est tel que défini ci-dessus, et on obtient un composé de formule (XXI); la réaction est conduite dans un solvant tel que le chloroforme en présence d'une base comme la triéthylamine.
Dans une septième étape, on réalise la déprotection du noyau imidazolé du composé (XXI) soit par hydrogénation, soit par action de l'acide acétique dans un mélange tétrahydrofurane/eau. On obtient un composé de formule (I ter). Lorsqu'on hydrogène, avec chauffage, un composé de formule (XXI) dans laquelle Ar représente le groupe 3-méthyl-quinoléin-8-yle, on obtient des composés de formule (I ter) dans laquelle Ar est le groupe 3-méthyl-1,2,3,4-tétrahydroquinoléin-8-yle.
Lorsque R₂ est un groupe (C₁-C₄)alcoxycarbonyle, on peut faire réagir le composé (I ter) avec une solution d'hydroxyde de sodium pour obtenir le sel de l'acide correspondant.

Les composés de l'invention pour lesquels X représente un atome d'oxygène répondent à la formule (I quater) 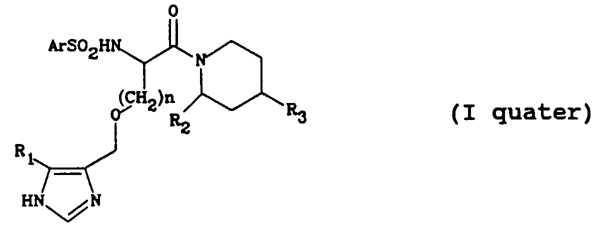 dans laquelle n = 1 ou 2 et R₁, R₂, R₃ et Ar sont tels que définis précédemment; ils peuvent être synthètisés selon le schéma 4 de la page suivante.
Dans une première étape, on fait réagir un alcool de formule (II) avec du chlorure de thionyle dans un mélange de diméthylformamide et de dichlorométhane; ensuite on fait réagir le composé obtenu avec un composé de formule (XXII) préalablement traité en présence d'un excès d'hydrure de sodium dans le tétrahydrofurane et on obtient le composé de formule (XXIII).
Dans une seconde étape, on condense le composé de formule (XXIII) avec un composé de formule (IX) et on obtient un composé de formule (XXIV); la réaction est réalisée en présence d'une base comme la 4-méthylmorpholine ou la N,N-diisopropyléthylamine (base de "Hunig") et d'un agent couplant tel que l'hexafluorophosphate de [(benzotriazol-1-yl)oxy]tris(diméthylamino)phosphonium, le diphénylphosphorylazide ou le chlorhydrate de 1-(3-diméthylaminopropyl)-3-éthylcarbodiimide couplé avec le 1-hydroxybenzotriazole.
Dans une troisième étape, on réalise la déprotection de la fonction amine primaire par action de l'acide trifluoracétique dans un mélange de dichloroéthane et de méthanol, puis on fait réagir un chlorure d'arysulfonyle de formule ArSO₂Cl, dans laquelle Ar est tel que défini précédemment, dans un solvant tel que le chloroforme ou le dichlorométhane en présence d'une base comme la triéthylamine.
Enfin dans une dernière étape on déprotège le noyau imidazolé du composé de formule (XXIV), par action de l'acide acétique dans un mélange de tétrahydrofurane et d'eau pour obtenir un composé de formule (I quater).

Le brevet européen 0008746 décrit des dérivés de N²-arylsulfonyl-*L*-argininamide.

Les composés de départ sont disponibles dans le commerce ou décrits dans la littérature ou peuvent être préparés selon des méthodes qui y sont décrites ou qui sont connues de l'homme du métier.

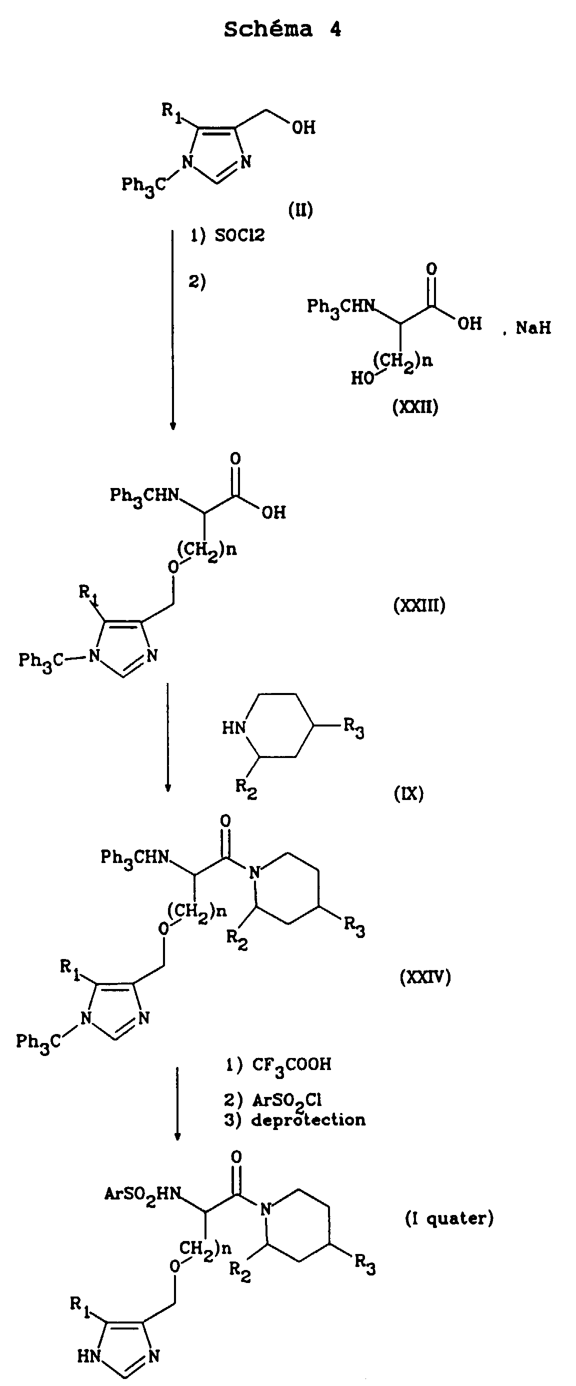

Ainsi les composés de formule (II) sont préparés par des méthodes analogues à celles décrites dans le brevet européen 0242973 et à celles décrites par Griffith R.K. et coll., Synthesis, 1983, 576.
Certains composés de formule (IV) sont décrits par Valerio R.M. et coll. dans Synthesis, 1988, 786.
La préparation de la 8-(chlorosulfonyl)-3-méthylquinoléine est décrite dans le brevet japonais 59184161.
La 8-(chlorosulfonyl)-3-méthyl-1,2,3,4-tétrahydroquinoléine peut être préparée à partir de la 8-(chlorosulfonyl)-3-méthyl-quinoléine de la manière suivante : on réalise une hydrolyse de la 8-(chlorosulfonyl)-3-méthylquinoléine par une solution aqueuse d'hydroxyde de sodium, puis après acidification du sulfonate de sodium ainsi obtenu, on réalise une hydrogènation catalytique en présence d'un catalyseur au rhodium. Ensuite, on traite le composé obtenu sous forme de sel de triéthylammonium par le complexe triphénylphosphinechlorure de sulfurylé dans un solvant tel que le dichlorométhane.
La préparation des composés de formule (IX) dans laquelle R₂ est un groupe carboxylique ou éthoxycarbonyle et R₃ un groupe méthyle ou éthyle est décrite dans le brevet européen 0008746. Après résolution chimique à l'acide tartrique, on obtient ces composés sous la forme [2R,4R] et on les utilise sous cette forme pour préparer les autres composés de formule (IX).

Ainsi les composés de formule (IX) dans laquelle R₂ représente un groupe -CH₂OR₄ où R₄ est un atome d'hydrogène peuvent être synthètisés de la manière suivante : on traite un composé de formule (IX) dans laquelle R₂ est un groupe éthoxycarbonyle par du dicarbonate de bis(1,1-diméthyléthyle) dans un solvant tel que le dichlorométhane dans des conditions classiques. On obtient un composé dont on réduit la fonction carboxyle, soit par la méthode à l'anhydride mixte décrite par Valerio R.M. et coll. dans Synthesis, 1988, 786, soit par l'action du diméthylsulfure de borane dans un solvant tel que le tétrahydrofurane. Ensuite, on déprotège directement l'amine par action de l'acide trifluoracétique et on obtient un composé de formule (IX) dans laquelle R₂ représente un groupe -CH₂OR₄ où R₄ est un atome d'hydrogène, sous forme de sel directement utilisable.
Les composés de formule (IX) dans laquelle R₂ représente un groupe -CH₂OR₄ où R₄ est un groupe (C₁-C₄)alkyle peuvent être synthètisés de la manière suivante : on fait réagir un composé de formule (IX) dans laquelle R₂ représente un groupe -CH₂OR₄ où R₄ est un atome d'hydrogène, en présence d'une base tel que l'hydrure de sodium avec un halogènure d'alkyle de formule R₄-X dans laquelle R₄ est un un groupe (C₁-C₄)alkyle et X est un atome d'halogène, de préférence un atome d'iode, avant de réaliser la déprotection par action de l'acide trifluoracétique dans le dichlorométhane et on obtient un composé de formule (IX) dans laquelle R₂ représente un groupe -CH₂OR₄ où R₄ est un groupe (C₁-C₄)alkyle.
Les composés de formule (IX) dans laquelle R₂ représente un groupe -CH₂OR₄ où R₄ est un groupe (C₁-C₄)acyle peuvent être préparés directement à partir des composés de formule (IX) dans laquelle R₂ représente un groupe -CH₂OR₄ où R₄ est un atome d'hydrogène, par réaction avec un chlorure de (C₁-C₄)acyle, en présence d'une base comme la triéthylamine.
La déprotection de l'amine est effectuée par action de l'acide trifluoroacétique; on obtient le composé sous forme de sel directement utilisable.
Ainsi les composés de formule (IX) dans laquelle R₂ représente un groupe -CONR₅R₆ où R₅ est un atome d'hydrogène ou un groupe (C₁-C₄)alkyle et R₆ est un atome d'hydrogène, un groupe (C₁-C₄)alkyle, un groupe hydroxy ou un groupe (C₁-C₄)alcoxy peuvent être préparés de la manière suivante : on traite un composé de formule (IX) dans laquelle R₂ est un groupe éthoxycarbonyle par du dicarbonate de bis(1,1-diméthyléthyle), dans un solvant tel que le dichlorométhane, dans des conditions classiques. On obtient un composé que l'on saponifie par une solution aqueuse d'hydroxyde de sodium puis on fait réagir une amine de formule HNR₅R₆ dans laquelle R₅ et R₆ sont tels que définis précédemment pour obtenir un amide; la réaction est réalisée en présence d'une base comme la 4-méthylmorpholine ou la N,N-diisopropyléthylamine (base de "Hunig") et d'un agent couplant tel que l'hexafluorophosphate de [(benzotriazol-1-yl)oxy]tris(diméthylamino)phosphonium ou l'isobutylchloro-formiate. On déprotège le groupe amine par action de l'acide trifluoracétique dans le dichlorométhane, éventuellement en présence de méthoxybenzène et on obtient les composés sous forme de sels de trifluoroacétate qui sont utilisables directement.

Les composés de formule (IX) dans laquelle R₂ représente un groupe -CN₄R₅ dans lequel R₅ est un atome d'hydrogène peuvent être synthètisés de la manière suivante : on traite un composé de formule (IX) dans laquelle R₂ est un groupe éthoxycarbonyle par du dicarbonate de bis(1,1-diméthyléthyle) dans un solvant tel que le dichlorométhane dans des conditions classiques. On obtient un composé que l'on condense avec de l'ammoniaque; la réaction est réalisée en présence d'une base comme la 4-méthylmorpholine et d'un agent couplant tel que l'isobutylchloroformiate dans un solvant tel que le tétrahydrofurane. On obtient ainsi un carboxamide que l'on déshydrate par de l'oxytrichlorure de phosphore pour obtenir un nitrile que l'on chauffe en présence d'azoture de sodium et de chlorure d'ammonium dans un solvant tel que le diméthylformamide. On obtient un dérivé tétrazolylé que l'on traite par l'acide trifluoracétique et on obtient un composé de formule (IX) dans laquelle R₂ représente un groupe -CN₄R₅ où R₅ est un atome d'hydrogène.
Les composés de formule (IX) dans laquelle R₂ représente un groupe -CN₄R₅ dans lequel R₅ est un groupe (C₁-C₄)alkyle peuvent être synthètisés de la manière suivante : on condense un composé de formule (IX) dans laquelle R₂ représente un groupe -CN₄R₅ où R₅ est un atome d'hydrogène avec un halogènure d'alkyle de formule R₅-X dans laquelle R₅ est un un groupe (C₁-C₄)alkyle et X est un atome d'halogène, de préférence un atome d'iode, avant de réaliser la déprotection par action de l'acide trifluoracétique dans le dichlorométhane et on obtient les composés de formule (IX) dans laquelle R₂ représente un groupe -CN₄R₅ où R₅ est un groupe (C₁-C₄)alkyle.

Les composés de formule (XXII) sont décrits par Barlos et coll dans J. Org. Chem., 1982, 47, 1324 et traités comme il est décrit par Barlos et coll. dans Tetrahedron, 1983, 39, 475.

Les exemples suivants illustrent la préparation de certains composés conformément à l'invention.
Les microanalyses élémentaires confirment la structure des composés obtenus.

### Exemple 1

[2*R*-[1(*S*),2α,4β]]-4-méthyl-1-[2-[[(3-méthyl-1,2,3,4-tétrahydroquinoléin-8-yl)sulfonyl]amino]-1-oxo-5-(1*H*-imidazol-4-yl)pentyl]pipéridine-2-carboxylate d'éthyle

### 1.1. chlorure de triphényl[[(1-triphénylméthyl)-1H-imidazol-4-yl]méthyl]phosphonium

A 670 ml d'une solution de 105,5 g (294 mmoles) de 4-(chlorométhyl)-1-(triphénylméthyl)-1*H*-imidazole dans le diméthylformamide, on ajoute 77,7 g (296 mmoles) de triphénylphosphine. On chauffe à 80 °C pendant 3 heures. On évapore le solvant, on reprend le brut dans l'éther et on le triture. On filtre le précipité et on le sèche sous'vide sur du pentoxyde de phosphore.
On obtient 162 g de produit sous forme de cristaux jaunâtres.
Point de fusion = 210 °C Rendement = 89 %

### 1.2. (S,E)-2-[[(phénylméthoxy)carbonyl]amino]-5-[1-(triphénylméthyl)-1H-imidazol-4-yl]pent-4-énoate de 1,1-diméthyléthyle

Dans un ballon tricol, sous argon, on introduit 50,93 g (820 mmoles) de chlorure de triphényl[[1-triphénylméthyl)-1*H*-imidazol-4-yl]méthyl]phosphonium en solution dans 333 ml de tétrahydrofurane. On ajoute goutte à goutte, à - 70 °C, 51,2 ml d'une solution 1,6 M de n-butyllithium dans l'hexane (820 mmoles). Aprés 30 minutes d'agitation à - 70 °C, on verse rapidement le milieu réactionnel dans 270 ml d'une solution 0,253 M, refroidie à 70 °C, de (*S*)-4-oxo-2-[[(phénylméthoxy)-carbonyl]amino]butanoate de 1,1-diméthyléthyle dans le tétrahydrofurane (683 mmoles). On laisse remonter le mélange à la température ambiante pendant une nuit. On hydrolyse le mélange avec 280 ml d'une solution aqueuse saturée de chlorure de sodium. On sépare la phase aqueuse de la phase organique et on l'extrait par 2 fois 140 ml d'acétate d'éthyle. On réunit les phases organiques, on les sèche sur du sulfate de magnésium et on évapore à sec. On purifie par chromatographie sur colonne de gel de silice en éluant par un gradient hexane/acétate d'éthyle.
On obtient un mélange d'oléfine cis et trans.
Pour la forme cis:
Point de fusion = 66 °C
R_{f} = 0,30 [hexane/acétate d'éthyle (60/40)]
Pour la forme trans:
R_{f} = 0,15 [hexane/acétate d'éthyle (60/40)]
Rendement = 40 %

### 1.3. chlorhydrate de (S)-2-amino-5-[1-(triphénylméthyl)-1H-imidazol-4-yl]pentanoate de 1,1-diméthyléthyle

On met en solution 5,83 g (9,50 mmoles) du composé cis obtenu précédemment en 1.2, dans 120 ml d'éthanol. On réalise, pendant 5 heures, une hydrogénation catalytique, à 3,5 bar (50 psi), en présence de palladium sur charbon comme catalyseur. On filtre le catalyseur sur un mélange célite/silice et on évapore le solvant. On recueille 4,32 g de produit que l'on dissout dans 90 ml d'alcool isopropylique "chlorhydrique" (HCl = 0,1 N) chaud. On évapore le solvant, on précipite par de l'éther et on sèche le produit sous vide.
On obtient 3,62 g de produit.
Point de fusion = 73 °C Rendement = 73 %

### 1.4. (S)-2-[[(3-méthylquinoléin-8-yl)sulfonyl]amino]-5-[1-(triphénylméthyl)-1H-imidazol-4-yl]pentanoate de 1,1-diméthyléthyle

A 3,8 g (7,26 mmoles) du composé obtenu précédemment en 1.3, on ajoute 1,76 g (7,28 mmoles) de 8-(chlorosulfonyl)-3-méthylquinoléine en solution dans 50 ml de chloroforme, en présence de 2,1 ml (14,5 mmoles) de triéthylamine, à 5°C. On laisse sous agitation pendant 3 heures, on sépare la phase organique, on la lave par une solution d'acide chlorhydrique 0,1 N et on évapore. On purifie le brut par chromatographie sur colonne de gel de silice en éluant par un mélange éthanol/dichlorométhane (5/95).
On obtient 3,6 g de produit.
Point de fusion = 56 °C Rendement = 72 %

### 1.5. acide (S)-2-[[(3-méthylquinoléin-8-yl)sulfonyl]amino]-5-[1-(triphénylméthyl)-1H-imidazol-4-yl]pentanoïque

Dans une solution de 2,33 g (3,39 mmoles) du composé obtenu précédemment en 1.4, dans 34 ml de benzène, refroidie à 0 °C, sous azote, on fait barboter, pendant 15 minutes, de l'acide chlorhydrique gazeux. On laisse le milieu réactionnel revenir à la température ambiante et on maintient l'agitation pendant 2 heures à cette température. On évapore le solvant sous vide et on purifie le résidu par chromatographie sur colonne de gel de silice en éluant par un mélange éthanol/dichlorométhane (20/80).
On obtient 1,42 g de produit sous forme d'une poudre blanchâtre.
Point de fusion = 170 °C Rendement = 66 %

### 1.6. [2R-[1(S),2α,4β]]-4-méthyl-1-[2-[[(3-méthylquinoléin-8-yl)sulfonyl]amino]-1-oxo-5-[1-(triphénylméthyl)-1H-imidazol-4-yl]pentyl]pipéridine-2-carboxylate d'éthyle

A une suspension de 2,23 g (3,53 mmoles) du composé précédemment obtenu en 1.5 dans 50 ml d'acétonitrile, on ajoute, à 0°C, sous azote, 1,56 g (3,53 mmoles) d'hexafluorophosphate de [(benzotriazol-1-yl)oxy]tris(diméthylamino)phosphonium, 0,78 ml (7,06 mmoles) de 4-méthylmorpholine, 0,61 g (3,57 mmoles) de (2R-trans)-4-méthylpipéridine-2-carboxylate d'éthyle et 30 ml de dichlorométhane. On laisse, sous agitation, à la température ambiante, pendant 5 heures. On hydrolyse le milieu réactionnel par une solution saturée de chlorure de sodium et on l'extrait avec du chloroforme. On lave la phase organique par une solution d'acide chlorhydrique 0,1 N, puis par une solution saturée d'hydrogénocarbonate de sodium, puis par de l'eau et finalement par une solution de chlorure de sodium. On sèche sur du sulfate de magnésium. On purifie le brut obtenu par chromatographie sur colonne de gel de silice en éluant par un mélange éthanol/dichlorométhane (5/95).
On obtient 1,94 g de produit.
R_{f} = 0,68 (dichlorométhane/éthanol 95/5)
Rendement = 70 %

### 1.7. [2R-[1(S),2α,4β]]-4-méthyl-1-[2-[[(3-méthyl-1,2,3,4-tétrahydroquinoléin-8-yl)sulfonyl]amino]-1-oxo-5-(1H-imidazol-4-yl)pentyl]pipéridine-2-carboxylate d'éthyle

A 1,94 g (2,47 mmoles) de l'ester obtenu précédemment en 1.6, on ajoute 70 ml d'éthanol et 17 ml d'acide acétique. On réalise une hydrogénation catalytique, en présence de palladium sur charbon, à 80 °C, pendant 6 heures. On filtre le mélange et on évapore le solvant. On reprend le résidu obtenu par de l'acide chlorhydrique 1 N, on le lave par de l'éther et on l'extrait par de acétate d'éthyle.
On obtient 1,05 g de produit.
Point de fusion = 104 °C (chlorhydrate) Rendement = 78 %
[α]$\frac{\text{20}}{\text{D}}$ = + 101 (c = 0,2; méthanol)

### Exemple 2

complexe de sodium du [2*R*-[1(*S*),2α,4β]]-4-méthyl-1-[2-[[(3-méthyl-1,2,3,4-tétrahydroquinoléin-8-yl)sulfonyl]amino]-1- oxo-5-(1*H*-imidazol-4-yl)pentyl]pipéridine-2-carboxylate de sodium

A une solution de 0,201 g (0,4 mmole) d'ester obtenu précédemment dans l'exemple 1, on ajoute, à la température ambiante, sous argon, 0,4 ml d'une solution de soude à 1 N titrée. On agite à la température ambiante pendant 36 heures et on suit la réaction par chromatographie sur couche mince. Lorsque la réaction est terminée, on chauffe le milieu réactionnel à 55 °C pendant 7 heures. On laisse refroidir et on ajoute 1 ml d'éthanol et 50 ml d'éther. Après trituration, on obtient un précipité blanchâtre que l'on filtre et que l'on sèche.
On obtient 170 mg de produit sous forme d'une poudre blanche.
Point de fusion = 215 °C Rendement = 86 %
[α]$\frac{\text{20}}{\text{D}}$= + 67,2 (c = 0,1; méthanol)

### Exemple 3

chlorhydrate de (*R*)-1-[2-[[[5-(diméthylamino)naphtalén-1-yl]sulfonyl]amino]-1-oxo-3-[[[(1*H*-imidazol-4-yl)méthyl]thio]propyl]-4-éthyl-pipéridine

### 3.1. acide 2-amino-3-[[[1-(triphénylméthyl)-1H-imidazol-4-yl]méthyl]thio]propanoïque

A 40 ml d'une solution aqueuse 1 N d'hydroxyde de sodium (40 mmoles), on ajoute 2,66 g (22 mmoles) de *L*-cystéine. On agite et on ajoute, sous agitation, à 0 °C, une solution de 7,2 g de 4-(chlorométhyl)-1-(triphénylméthyl)-1*H*-imidazole (20 mmoles) dans 50 ml d'éthanol et 20 ml de tétrahydrofurane. On laisse remonter à la température ambiante et on agite pendant 1 heure. On évapore l'éthanol et le tétrahydrofurane sous pression réduite. On reprend le résidu dans 100 ml d'eau et 20 ml d'acide chlorhydrique 1 N. On filtre le précipité obtenu, on le lave à l'eau et on le sèche. On obtient 8 g de produit.
Point de fusion = 162-164 °C (décomposition)
Rendement = 90 %

### 3.2. acide (R)-2-[[[5-(diméthylamino)naphtalén-1-yl]sulfonyl]amino]-3-[[[1-(triphénylméthyl)-1H-imidazol-4-yl]méthyl]-thio]propanoïque

A une solution de 1,77 g (4 mmoles) du composé obtenu précédemment en 3.1, dans 20 ml d'hydroxyde de sodium 0,2 N refroidie dans un bain de glace, on ajoute, en une fois, sous forte agitation, 1,08 g (4 mmoles) de chlorure de 5-(diméthylamino)naphtalén-1-sulfonyle. On agite pendant 30 minutes. On ajoute 100 ml d'eau et on ajuste le pH à 2,5 avec une solution d'acide chlorhydrique 1 N. On essore le précipité visqueux obtenu et on le redissout dans du dichlorométhane. On sèche sur du sulfate de magnésium. On purifie le produit par chromatographie sur colonne de gel de silice en éluant par un mélange dichlorométhane/méthanol (95/5).
On obtient 1,2 g de produit.
Point de fusion = 128-130 °C Rendement = 50 %

### 3.3. (R)-1-[2-[[[5-(diméthylamino)naphtalén-1-yl]sulfonyl]amino]-1-oxo-3-[[[1-(triphénylméthyl)-1H-imidazol-4-yl]méthyl]thio]propyl]-4-éthylpipéridine

A l'abri de l'humidité, à 0 °C, on introduit dans 15 ml d'acétonitrile, 1 g (1,5 mmoles) du composé obtenu précédemment en 3.2, 0,22 g (1,5 mmoles) de chlorhydrate de 4-éthylpipéridine et 0,663 g (1,5 mmoles) d' hexafluorophosphate de [(benzotriazol-1-yl)oxy]tris(diméthylamino)phosphonium. On agite et on ajoute 0,34 ml (3,1 mmoles) de 4-méthylmorpholine. On agite pendant une nuit, on ajoute une solution saturée de chlorure de sodium et on extrait deux fois par de l'acétate d'éthyle. On lave les phases organiques par une solution d'acide chlorhydrique 1 N, puis par de l'eau, puis par une solution saturée d'hydrogénocarbonate de sodium puis par une solution saturée de chlorure de sodium. On sèche sur du sulfate de magnésium et on évapore le solvant sous pression réduite.
On obtient 1 g de produit que l'on utilise tel quel dans l'étape suivante.
Point de fusion = 99-100 °C Rendement = 87 %

### 3.4. chlorhydrate de (R)-1-[2-[[[5-(diméthylamino)naphtalén-1-yl]sulfonyl]amino]-1-oxo-3-[[[(1H-imidazol-4-yl)méthyl]thio]propyl]-4-éthyl-pipéridine

On reprend, sous azote, 0,9 g (1,21 mmoles) du composé obtenu précédemment en 3.3, dans 20 ml d'acide acétique à 80 %. On chauffe, sous azote, pendant 20 minutes à la température de reflux et on évapore sous pression réduite. On purifie le résidu par chromatographie sur colonne de gel de silice en éluant par un mélange dichlorométhane/méthanol (90/10). On obtient 0,4 g de base.
On prépare le chlorhydrate par cristallisation dans un mélange isopropanol chlorhydrique/éther éthylique.
On obtient 0,15 g de produit.
Point de fusion = 105 °C (décompositon)
Rendement = 25 %
[α]$\frac{\text{20}}{\text{D}}$ = + 99 (c = 0,1; méthanol)

### Exemple 4

chlorhydrate de (*S*)-4-éthyl-1-[4-[[(1*H*-imidazol-4-yl)méthyl]thio]-2-[[(3-méthylquinoléin-8-yl)sulfonyl]amino]-1-oxobutyl]pipéridine

### 4.1. (S)-2-[[(phénylméthoxy)carbonyl]amino]-4-[[[1-(triphénylméthyl)-1H-imidazol-4-yl]méthyl]thio]butanoate de 1,1-diméthyléthyle

A une solution de 3,58 g (10 mmoles) de 4-(chlorométhyl)-1-(triphénylméthyl)-1*H*-imidazole dans 40 ml d'éthanol, sous azote, on ajoute 6 g de thioacétate de potassium. On agite par sonication pendant 15 minutes, puis on verse le milieu réactionnel sur de l'éther. On lave la phase organique à l'eau, puis par une solution saturée d'hydrogénocarbonate de sodium puis par une solution saturée de chlorure de sodium. On sèche sur du sulfate de magnésium et on évapore le solvant sous pression réduite. On reprend le résidu dans 100 ml de méthanol dégazé à l'azote et contenant 1,9 ml d'une solution 5,3 N de méthylate de sodium (10 mmoles). On agite pendant 15 minutes et on ajoute 4,63 g (10 mmoles) de (*S*)-4-[[(4-méthylphényl)sulfonyl]oxy]-2-[[(phénylméthoxy)carbonyl]amino]butanecarboxylate de 1,1-diméthyléthyle dans 40 ml de méthanol dégazé. On agite pendant 24 heures à la température ambiante. On essore le précipité obtenu, on le lave au méthanol et à l'eau. On le sèche sur du sulfate de magnésium.
On obtient 3 g de produit.
Point de fusion = 183-185 °C Rendement = 50 %

### 4.2. chlorhydrate de l'acide (S)-2-[[(phénylméthoxy)carbonyl]amino]-4-[[[1-(triphénylméthyl)-1H-imidazol-4-yl]méthyl]thio]butanoïque

On place 2,5 g (3,86 moles) du composé obtenu précédemment en 4.1 dans 50 ml de benzène. On sature la solution en acide chlorhydrique gazeux, à l'abri de l'humidité, à 0 °C, pendant 10 minutes. On agite le milieu réactionnel pendant 2 heures à 0 °C. On évapore sous pression réduite.
On obtient 2,2 g de produit que l'on utilisera tel quel pour la prochaine étape.
Point de fusion = 78-82 °C Rendement = 91 %

### 4.3. (S)-4-éthyl-1-[1-oxo-2-[[(phénylméthoxy)carbonyl]amino]-4-[[[1-(triphénylméthyl)-1H-imidazol-4-yl]méthyl]thio]butyl]pipéridine

On introduit, à l'abri de l'humidité, dans 15 ml d'acétonitrile, 0,627 g (1 mmole) du composé obtenu précédemment en 4.2, 0,149 g (1 mmole) de chlorhydrate de 4-éthylpipéridine, et 0,442 g (1 mmole) d'hexafluorophosphate de [(benzotriazol-1-yl)oxy]tris(diméthylamino)phosphonium. On agite et on ajoute, sous agitation, 0,33 ml (3 mmoles) de 4-méthylmorpholine. On agite pendant 5 heures à la température ambiante. On ajoute une solution saturée de chlorure de sodium et on extrait deux fois par de l'acétate d'éthyle. On réunit les phases organiques, on les lave par de l'eau, puis par une solution saturée d'hydrogénocarbonate de sodium puis par une solution saturée de chlorure de sodium. On sèche sur du sulfate de magnésium et on évapore le solvant sous pression réduite.
On obtient 0,7 g de produit sous forme d'une huile qui cristallise.
Point de fusion = 80-82 °C Rendement = 100 %

### 4.4. (S)-1-[2-amino-1-oxo-4-[[[1-(triphénylméthyl)-1H-imidazol-4-yl]méthyl]thio]butyl]-4-éthylpipéridine

Sous azote, à 10 °C, on place 0,7 g (1 mmole) du composé obtenu précédemment en 4.3, dans 1,2 ml d'acide acétique glacial. On ajoute, à l'abri de l'humidité, 3 ml d'une solution 4 N d'acide bromhydrique dans l'acide acétique. On laisse 1 heure, à l'abri de l'humidité et à la température ambiante, puis on verse le milieu réactionnel sur de l'éther. On décante le solide obtenu, on le lave plusieurs fois à l'éther et on le dissout dans 5 ml d'un mélange dichlorométhane/méthanol. On amène le mélange à pH basique par quelques gouttes d'ammoniaque aqueux concentré. On le purifie par chromatographie sur colonne de gel de silice en éluant par un mélange dichlorométhane/éthanol (90/10).
On obtient 240 mg de produit.
Point de fusion = 115-120 °C Rendement = 44 %

### 4.5. (S)-4-éthyl-1-[2-[[(3-méthylquinoléin-8-yl)sulfonyl]amino]-1-oxo-4-[[[1-(triphénylméthyl)-1H-imidazol-4-yl]méthyl]thio]butyl]pipéridine

On place 0,23 g (0,418 mmole) du composé obtenu précédemment en 4.4, dans 6 ml de dichlorométhane. On ajoute, sous agitation et à l'abri de l'humidité, 70 µl (0,5 mmole) de triéthylamine puis 110 mg (0,6 mmole) de 8-(chlorosulfonyl)-3-méthylquinoléine. On agite 30 minutes à la température ambiante. On dilue le milieu réactionnel avec de l'acétate d'éthyle. On lave avec une solution 1 N d'acide chlorhydrique, puis par de l'eau, puis par une solution saturée d'hydrogénocarbonate de sodium, puis par de l'eau, puis par une solution saturée de chlorure de sodium. On sèche sur du sulfate de magnésium et on évapore le solvant sous pression réduite.
On obtient 0,29 g de produit que l'on utilise tel quel dans l'étape suivante.
Point de fusion = 84-86 °C Rendement = 92 %

### 4.6. chlorhydrate de (S)-4-éthyl-1-[4-[[(1H-imidazol-4-yl)méthyl]thio]-2-[[(3-méthylquinoléin-8-yl)sulfonyl]amino]-1-oxobutyl]pipéridine

On place 0,28 g (0,37 mmole) du composé obtenu précédemment en 4.5, dans 10 ml d'une solution aqueuse d'acide acétique à 80 % et on ajoute 5 ml de tétrahydrofurane. On chauffe à la température de reflux, pendant 15 minutes, sous azote. On évapore sous pression réduite. On purifie le résidu par chromatographie sur colonne de gel de silice en éluant par un mélange dichlorométhane/méthanol. On obtient 0,16 g de produit sous forme de base.
On forme le chlorhydrate par cristallisation dans un mélange isopropanol/éther éthylique.
On obtient 0,14 g de produit sous forme de cristaux.
Point de fusion = 70-80 °C (décomposition)
Rendement = 66 %
[α]$\frac{\text{20}}{\text{D}}$ = + 127° (c = 0,1; méthanol)

### Exemple 5

[2*R*-[1(*S*), 2α, 4β]]-1-[3-[(1*H*-imidazol-4-yl)méthoxy]-2-[[(3-méthyl-1,2,3,4-tétrahydroquinoléin-8-yl)sulfonyl]amino]-1-oxopropyl]-4-méthylpipéridine-2-carboxylate d'éthyle

### 5.1. (N)-(triphénylméthyl)-(O)-[[1-(triphénylméthyl)-1H-imidazol-4-yl]méthyl]-(L)-sérine

A une suspension de 5,8 g (144,2 mmoles) d'hydrure de sodium à 60 % dans 112 ml de tétrahydrofurane, on ajoute, à - 15 °C, 0,39 g (5,7 mmoles) de 4-(chlorométhyl)-1-(triphénylméthyl)-1*H*-imidazole puis 10,01 g (28,83 mmoles) de (*N*)-(triphénylméthyl)-(*L*)-sérine. On laisse à -15 °C pendant 45 minutes et on ajoute 12 g (34,6 mmoles) de 4-(chlorométhyl)-1-(triphénylméthyl)-1*H*-imidazole. On agite pendant 2 heures à 0 °C puis pendant 5 heures à la température ambiante. On refroidit le milieu réactionnel à 0 °C, on dilue par 200 ml d'eau et on neutralise par addition de 6,59 ml (115 mmoles) d'acide acétique. On laisse décanter, on recueille la phase aqueuse et on l'extrait par 2 fois 200 ml d'acétate d'éthyle. On réunit les phases organiques, on les lave par environ 300 ml d'eau et on les sèche sur du sulfate de magnésium. On évapore les solvants et on purifie le résidu par chromatographie sur colonne de gel de silice en éluant par un gradient éthanol/dichlorométhane (2/98 à 4/96).
On obtient 12,82 g de produit.
Point de fusion = 104-106 °C Rendement = 68 %

### 5.2. 2-[[(3-méthyl-1,2,3,4-tétrahydroquinoléin-8-yl)sulfonyl]amino]-(O)-[[1-(triphénylméthyl)-1H-imidazol-4-yl]méthyl]-(L)-sérine

Sous azote, à la température ambiante, on place 1,34 g (2 mmoles) du composé précédemment obtenu en 5.1, dans 26 ml d'un mélange contenant 95,5 % de 1,2-dichloroéthane, 3 % de méthanol et 1,5 % d'acide trifluoroacétique. On laisse quelques minutes et on ajoute, à 0 °C, 0,84 ml (6 mmoles) de triéthylamine et 739 mg (3 mmoles) de chlorure de 3-méthyl-1,2,3,4-tétrahydroquinoléin-8-sulfonyle en solution dans 10 ml de chloroforme. On laisse agir pendant une heure puis on ajuste le pH à 3 avec de l'acide trifluoroacétique. On purifie par chromatographie sur colonne de gel de silice en éluant par un mélange chloroforme/méthanol (95/5). On obtient 1,14 g de produit.
Point de fusion = 145-150 °C Rendement = 91 %

### 5.3. [2R-[1(S), 2α, 4β]]-4-méthyl-1-[2-[[(3-méthyl-1,2,3,4-tétrahydroquinoléin-8-yl)sulfonyl]amino]-1-oxo-3-[[1-(triphénylméthyl)- 1H-imidazol-4-yl]méthoxy]propyl]pipéridine-2-carboxylate d'éthyle

On place, sous azote, à 0 °C, dans 15 ml de dichlorométhane, 1,2 g (1,94 mmoles) du composé obtenu précédemment en 5.2, 0,365 g (2,13 mmoles) de (2*R*-*trans*)-4-méthylpipéridine-2-carboxylate d'éthyle et 0,950 g (2,15 mmoles) d'hexafluorophosphate de [(benzotriazol-1-yl)oxy]tris(diméthylamino)phosphonium; on ajoute 0,5 ml (2,87 mmoles) de N,N-diisopropyléthylamine et on laisse agir une nuit à la température ambiante. Ensuite, on dilue par 100 ml d'acétate d'éthyle et on lave successivement par de l'acide chlorhydrique 1 N, par de l'eau puis par une solution saturée en hydrogénocarbonate de sodium et finalement par une solution saturée en chlorure de sodium. On sèche sur du sulfate de magnésium.
On récupère 1,2 g de produit que l'on utilise tel quel dans l'étape suivante.
Rendement = 80 %

### 5.4. [2R-[1(S), 2α, 4β]]-1-[3-[(1H-imidazol-4-yl)méthoxy]-2-[[(3-méthyl-1,2,3,4-tétrahydroquinoléin-8-yl)sulfonyl]amino]-1-oxopropyl]-4-méthylpipéridine-2-carboxylate d'éthyle, chlorhydrate

On place sous léger reflux, pendant 45 minutes, 1,2 g (1,55 mmoles) du composé obtenu précédemment en 5.3, dans 20 ml d'un mélange acide acétique/eau/tétrahydrofurane (50/10/40). On évapore et on purifie le mélange par chromatographie sur colonne de gel de silice en éluant par un mélange méthanol/dichlorométhane (5/95 à 10/90).
On obtient 0,6 g de produit sous forme de base.
On prépare le chlorhydrate en mettant 300 mg de base dans 6 ml d'un mélange d'acide chlorhydrique 0,1 N dans l'alcool isopropylique et d'éther diéthylique. On essore et on décante.
On obtient 180 mg de chlorhydrate.
Point de fusion = 62-64 °C Rendement = 56 %
[α]$\frac{\text{20}}{\text{D}}$ = + 71 (c = 0,1; méthanol)

### Exemple 6

chlorhydrate de (2*R*-*trans*)-*N*-éthyl-1-[5-(1*H*-imidazol-4-yl)-2-[[(3-méthyl-1,2,3,4-tétrahydroquinoléin-8-yl)sulfonyl] amino]-1-oxopentyl]-4-méthylpipéridine-2-carboxamide

### 6.1. trifluoroacétate de (2R-trans)-N-éthyl-4-méthylpipéridine-2-carboxamide

### 6.1.1. (2R-trans)-2-[(éthylamino)carbonyl]-4-méthylpipéridine-1-carboxylate de 1,1-diméthyléthyle

On dissout 1 g (4,11 mmoles) de (2*R*-*trans*)-4-méthylpipéridine-1,2-dicarboxylate de 1-(1,1-diméthyléthyle) dans 15 ml de tétrahydrofurane. Sous argon et à la température ambiante, on ajoute 0,45 ml (4 mmoles) de 4-méthylmorpholine puis on refroidit à -10 °C -15 °C. On ajoute ensuite 0,53 ml (4 mmoles) de chloroformate de 2-méthylpropyle puis 0,278 g (6 mmoles) d'éthanamine en solution dans 3 ml de tétrahydrofurane. On laisse remonter le milieu à la température ambiante puis on évapore le tétrahydrofurane. On reprend le résidu par 100 ml d'éther et on le lave successivement avec 50 ml d'acide chlorhydrique 1 N, 50 ml d'eau et finalement 50 ml d'une solution saturée en hydrogénocarbonate de sodium. On sèche sur du sulfate de magnésium.
On obtient 1,06 g de produit.
Rendement = 95 %

### 6.1.2. trifluoroacétate de (2R-trans)-N-éthyl-4-méthylpipéridine-2-carboxamide

On met en solution dans 5,4 ml de dichlorométhane, 0,352 g (1,3 mmoles) du composé obtenu précedemment en 6.1. Sous argon et à 0 °C, on ajoute 5,4 ml d'acide trifluoroacétique. On laisse le milieu remonter à la température ambiante puis on évapore. On reprend le résidu dans 50 ml de dichlorométhane et on évapore de nouveau.
On obtient 0,686 g de produit brut.

### 6.2. acide (S)-α-[[(3-méthyl-1,2,3,4-tétrahydroquinoléin-8-yl)sulfonyl]amino]-1-(triphénylméthyl)-1H-imidazole-4-pentanoïque

### 6.2.1. acide 3-méthyl-1,2,3,4-tétrahydroquinoléine-8-sulfonique

On place 20 g (90 mmoles) d'acide 3-méthylquinoléine-8-sulfonique dans un mélange contenant 200 ml d'eau et 25 ml d'acide chlorhydrique 12 N. On ajoute 6 g de catalyseur au rhodium à 5 % et on chauffe à 70 °C pendant 16 heures. On rince le catalyseur à l'eau chaude et on évapore. On reprend le résidu par 50 ml d'éthanol et on le sèche sur du pentoxyde de phosphore, à 50 °C.
On obtient 14 g de produit.
Point de fusion = 255 °C (décomposition)

### 6.2.2. (S)-α-[[(3-méthyl-1,2,3,4-tétrahydroquinoléin-8-yl)sulfonyl]amino]-1-(triphénylméthyl)-1H-imidazole-4-pentanoate d'éthyle

On dissout 10,46 g (40 mmoles) de triphénylphosphine dans 60 ml de dichlorométhane, à 0 °C. Sous azote, à 0 °C, on ajoute goutte à goutte 3,56 ml (44 mmoles) de chlorure de sulfurylé. Ensuite, on laisse remonter à la température ambiante et on ajoute, en 10 minutes, 4,56 g (20 mmoles) du composé précédemment obtenu en 6.2.1 dissous dans un mélange contenant 100 ml de dichlorométhane et 2,78 ml de triéthylamine. On laisse sous agitation pendant environ une heure à la température ambiante puis on verse le milieu réactionnel sur 500 ml de pentane. On filtre, on évapore le filtrat et on reprend le résidu par environ 500 ml de pentane. On obtient 5 g de produit sous forme d'une huile que l'on reprend dans 50 ml de dichlorométhane. Sous azote, à 0 °C, on ajoute 35 ml de cette dernière solution à une solution de 4 g (7,72 mmoles) de chlorhydrate de (*S*)-α-amino-1-(triphénylméthyl)-1*H*-imidazole-4-pentanoate d'éthyle dans 50 ml de dichlorométhane. On laisse sous agitation pendant une heure puis on lave successivement par 50 ml d'acide chlorhydrique 1 N, 50 ml d'eau, 50 ml d'une solution saturée en hydrogénocarbonate de sodium, 50 ml d'eau et finalement par 50 ml d'une solution saturée en chlorure de sodium. On sèche sur du sulfate de magnésium et on purifie le résidu par chromatographie sur colonne de gel de silice en éluant par un mélange dichlorométhane/méthanol (97/3).
On obtient 4,7 g de produit.

Point de fusion = 70-80 °C Rendement = 88 % (par rapport à l'amine)

### 6.2.3. acide (S)-α-[[(3-méthyl-1,2,3,4-tétrahydroquinoléin-8-yl)sulfonyl]amino]-1-(triphénylméthyl)-1H-imidazole-4-pentanoïque

On place 4,5 g (6,5 mmoles) du composé précédemment obtenu en 6.2.2 dans 80 ml de benzène, on refroidit à 0 °C et on laisse barboter pendant une heure de l'acide chlorhydrique gazeux. On laisse ensuite encore 30 minutes à 0 °C et on évapore. On reprend le résidu dans 10 ml de chloroforme et on ajuste le pH à 8-9 avec de l'ammoniac en solution dans du dichlorométhane. On purifie par chromatographie sur colonne de gel de silice en éluant par un gradient dichlorométhane/méthanol (95/5 à 90/10).
On obtient 3,5 g de produit que l'on utilise tel quel dans l'étape suivante.
Point de fusion = 125-145 °C Rendement = 85 %

### 6.3. (2R-trans)-N-éthyl-4-méthyl-1-[2-[[(3-méthyl-1,2,3,4-tétrahydroquinoléin-8-yl)sulfonyl]amino]-1-oxo-5-[1-(triphénylméthyl)- 1H-imidazol-4-yl]pentylpipéridine-2-carboxamide

On met en solution 0,221 g (1,3 mmoles) du composé précédemment obtenu en 6.1, dans 10 ml de dichlorométhane. Sous argon et à 0 °C, on ajoute 0,635 g (1 mmole) du composé précédemment obtenu en 6.2, 0,486 g (1,10 mmoles) d'hexafluorophosphate de [(benzotriazol-1-yl)oxy]tris(diméthylamino)phosphonium et 1,04 ml (6 mmoles) de N,N-diisopropyléthylamine. On laisse le mélange remonter à la température ambiante pendant une nuit puis on lave successivement par 10 ml d'acide chlorhydrique 1 N et par 10 ml d'une solution saturée en hydrogénocarbonate de sodium. On sèche sur du sulfate de magnésium.
On obtient 0,337 g de produit.
Point de fusion = 30-40°C Rendement = 43 %

### 6.4. chlorhydrate de (2R-trans)-N-éthyl-1-[5-(1H-imidazol-4-yl)-2-[[(3-méthyl-1,2,3,4-tétrahydroquinoléin-8-yl)sulfonyl]amino]-1-oxopentyl]-4-méthylpipéridine-2-carboxamide

On solubilise 0,332 g (0,422 mmole) du composé précédemment obtenu en 6.3, dans 3,7 ml d'acide acétique, puis on ajoute 0,7 ml d'eau et 3 ml de tétrahydrofurane. On chauffe le mélange au reflux pendant une heure. On évapore à sec et on purifie par chromatographie sur colonne de gel de silice en éluant par un gradient dichlorométhane/éthanol (90/10 à 85/15).
On obtient 0,225 g de produit sous forme de base. On prépare le chlorhydrate en mettant 0,225 g de base en solution dans 4,1 ml d'acide chlorhydrique 0,1 N dans l'alcool isopropylique. On évapore et on reprend le résidu par de l'éther. On filtre et on sèche.
On obtient 0,200 g de chlorhydrate.
Point de fusion = 105-110 °C (décomposition)
[α]$\frac{\text{20}}{\text{D}}$ = + 33,2 (c = 0,2; méthanol)

### Exemple 7

chlorhydrate de [2*R*-[1(*S*), 2α, 4β]]-*N*-hydroxy-1-[5-(1*H*-imidazol-4-yl)-2-[[(3-méthyl-1,2,3,4-tétrahydroquinoléin-8-yl)sulfonyl]amino]- 1-oxopentyl]-4-méthylpipéridine-2-carboxamide

### 7.1. trifluoroacétate de (2R-trans)-4-méthyl-N-(phénylméthoxy)pipéridine-2-carboxamide

### 7.1.1. acide (2R-trans)-1-(2,2-diméthyl-1-oxopropyl)-4-méthylpipéridine-2-carboxylique

On place 0,51 g (3 mmoles) de (2*R*-*trans*)-4-méthylpipéridine-2-carboxylate d'éthyle dans 10 ml de dichlorométhane et on ajoute, sous azote, à 0 °C, 654 mg (3 mmoles) de dicarbonate de bis(1,1-diméthyléthyle). On laisse remonter à la température ambiante pendant une nuit. On évapore et on reprend le résidu dans 4 ml d'éthanol et 3 ml d'une solution d'hydroxyde de sodium 1 N. On laisse une nuit à la température ambiante puis on ajoute de nouveau 3 ml d'une solution d'hydroxyde de sodium 1 N. Ensuite, on évapore, on reprend le résidu par de l'eau et on lave avec de l'éther. On acidifie le milieu avec de l'acide chlorhydrique 1 N puis on extrait deux fois par de l'éther. On rince les phases organiques par 10 ml d'une solution saturée en chlorure de sodium et on sèche sur du sulfate de magnésium.
On obtient 0,68 g de produit.
Point de fusion = 87-90 °C Rendement = 97 %

### 7.1.2. (2R-trans)-1-(2,2-diméthyl-1-oxopropyl)-4-méthyl-N-(phénylméthoxy)pipéridine-2-carboxamide

Sous azote, on place 1 g (4,03 mmoles) du composé précédemment obtenu en 7.1.1, dans 15 ml de tétrahydrofurane. On refroidit à -15 °C et on ajoute 0,47 ml (4 mmoles) de 4-méthylmorpholine et 0,56 ml (4 mmoles) de chloroformate de 2-méthylpropyle. On agite pendant 5 minutes et on ajoute 0,755 g (4,7 mmoles) de chlorhydrate de *O*-(phénylméthyl)hydroxylamine, en solution dans un mélange de 5 ml de tétrahydrofurane et de 0,52 ml de 4-méthylmorpholine. On agite pendant une nuit à la température ambiante puis on verse le milieu réactionnel sur 3 volumes d'acétate d'éthyle. On lave successivement par 50 ml d'acide chlorhydrique 0,1 N, 50 ml d'eau, 50 ml d'hydrogénocarbonate de sodium, 50 ml d'eau et finalement par 50 ml d'une solution saturée en chlorure de sodium. On séche sur du sulfate de magnésium et on recristallise le résidu par trituration dans l'éther.
On obtient 0,95 g de produit.
Point de fusion = 107-108 °C

### 7.1.3. trifluoroacétate de (2R-trans)-4-méthyl-N-(phénylméthoxy)pipéridine-2-carboxamide

On agite pendant 15 minutes, à 0 °C, une solution de 450 mg (1,4 mmoles) du composé précédemment obtenu en 7.1.2, dans un mélange de 5 ml d'acide trifluoroacétique et de 5 ml de dichlorométhane. On évapore, on reprend le résidu par 10 ml de benzène et on évapore de nouveau.
On obtient 700 mg de produit sous forme d'une huile utilisée telle quelle dans l'étape suivante.

### 7.2. chlorhydrate de [2R-[1(S), 2α, 4β]]-N-hydroxy-1-[5-(1H-imidazol-4-yl)-2-[[(3-méthyl-1,2,3,4-tétrahydroquinoléin-8-yl)sulfonyl]amino]- 1-oxopentyl]-4-méthylpipéridine-2-carboxamide

### 7.2.1. [2R-[1(S), 2α, 4β]]-4-méthyl-1-[2-[[(3-méthyl-1,2,3,4-tétrahydroquinoléin-8-yl)sulfonyl]amino]-1-oxo-5-[1-(triphénylméthyl)-1H-imidazol-4- yl]pentyl]-N-(phénylméthoxy)pipéridine-2-carboxamide

On reprend dans 10 ml de dichlorométhane 700 mg (1,4 mmoles) du composé précédemment obtenu en 7.1 et on refroidit le mélange à 0 °C. Sous azote, on ajoute 630 mg (1 mmole) du composé précédemment obtenu en 6.2, 500 mg (1,13 mmoles) d'hexafluorophosphate de [(benzotriazol-1-yl)oxy]tris(diméthylamino)phosphonium et 0,875 ml (5 mmoles) de N,N-diisopropyléthylamine. On laisse sous agitation, pendant une nuit, à la température ambiante puis on verse le mileu réactionnel sur 80 ml d'acétate d'éthyle. On lave successivement par 80 ml d'acide chlorhydrique 0,1 N, 80 ml d'eau, 80 ml d'une solution d'hydrogénocarbonate de sodium, et finalement par 80 ml d'une solution saturée en chlorure de sodium. On sèche sur du sulfate de magnésium et on purifie le résidu par chromatographie sur colonne de gel de silice en éluant par un mélange dichlorométhane/méthanol (97/3).
On obtient 0,8 g de produit.
Point de fusion = 90-100 °C Rendement = 93 %

### 7.2.2. [2R-[1(S), 2α, 4β]]-1-[5-(1H-imidazol-4-yl)-2-[[(3-méthyl-1,2,3,4-tétrahydroquinoléin-8-yl)sulfonyl]amino]-1-oxopentyl]-4- méthyl-N-(phénylméthoxy)pipéridine-2-carboxamide

Sous azote, on chauffe au reflux pendant une heure, 0,8 g (0,93 mmole) du composé précédemment obtenu en 7.2.1 dans 10 ml de tétrahydrofurane et 10 ml d'acide acétique à 80 %. On évapore, on reprend le résidu par 10 ml de dichlorométhane et on le purifie par chromatographie sur colonne de gel de silice en éluant par un mélange de dichlorométhane/méthanol (90/10).
On obtient 0,5 g de produit.
Point de fusion = 75-95 °C Rendement = 88 %

### 7.2.3. chlorhydrate de [2R-[1(S), 2α, 4β]]-N-hydroxy-1-[5-(1H-imidazol-4-yl)-2-[[(3-méthyl-1,2,3,4-tétrahydroquinoléin-8-yl)sulfonyl]amino]-1- oxopentyl]-4-méthylpipéridine-2-carboxamide

Dans une fiole de Parr, on place 0,45 g (0,73 mmole) du composé précédemment obtenu en 7.2.2 dans 30 ml de méthanol. On ajoute 0,1 g de catalyseur au palladium sur carbone à 10 % et on hydrogène pendant 10 heures à 2,8 bar (40 psi). On essore le catalyseur, on évapore et on purifie le résidu par chromatographie sur colonne de gel de silice en éluant par un mélange dichlorométhane/méthanol (90/10).
On obtient 0,2 g de produit sous forme de base.
On prépare le chlorhydrate en précipitant le produit sous forme de base dans un mélange d'isopropanol et d'éthanol. On obtient 0,15 g de chlorhydrate.
Point de fusion = 140 °C (décomposition)
[α]$\frac{\text{20}}{\text{D}}$ = + 85 (c = 0,1; méthanol)

### Exemple 8

chlorhydrate de [2*R*-[1(*S*), 2α, 4β]]-1-[5-(1*H*-imidazol-4-yl)-2-[[(3-méthyl-1,2,3,4-tétrahydroquinoléin-8-yl)sulfonyl]amino]-1-oxopentyl]- 4-méthyl-2-(1*H*-tétrazol-4-yl)pipéridine

### 8.1. trifluoroacétate de (2R-trans)-4-méthyl-2-(1H-tétrazol-4-yl)pipéridine

### 8.1.1. (2R-trans)-1-(2,2-diméthyl-1-oxopropyl)-4-méthylpipéridine-2-carboxamide

Sous azote, on place 1 g (4,03 mmoles) du composé précédemment obtenu en 7.1.1, dans 15 ml de tétrahydrofurane. On refroidit le milieu réactionnel à -15 °C et on ajoute 0,47 ml (4 mmoles) de 4-méthylmorpholine puis 0,56 ml (4 mmoles) de chloroformate de 2-méthylpropyle. On agite pendant 5 minutes puis on ajoute en excès de l'ammoniac. On laisse remonter à la température ambiante puis on agite pendant 1 heure. On verse le milieu réactionnel sur 3 volumes d'acétate d'éthyle et on lave successivement par 50 ml d'acide chlorhydrique 0,1 N, 50 ml d'eau, 50 ml d'une solution d'hydrogénocarbonate de sodium, 50 ml d'eau et finalement par 50 ml d'une solution saturée en chlorure de sodium. On sèche sur du sulfate de magnésium et on évapore.
On obtient 1 g de produit sous forme d'une huile opaque que l'on utilise telle quelle dans l'étape suivante.

### 8.1.2. (2R-trans)-1-(2,2-diméthyl-1-oxopropyl)-4-méthylpipéridine-2-carbonitrile

On reprend 1 g (4 mmoles) du composé précédemment obtenu en 8.1.1 dans 5 ml de pyridine, on refroidit à -5 °C et on ajoute goutte à goutte, 0,52 ml de chlorure de phosphoryle dans 1 ml de dichlorométhane. On agite pendant 1 heure puis on verse le milieu réactionnel sur de la glace et on extrait par 100 ml d'éther diéthylique. On évapore et on purifie le résidu par chromatographie sur colonne de gel de silice en éluant par du dichlorométhane.
On obtient 0,7 g de produit sous forme d'une huile que l'on utilise telle quelle dans l'étape suivante.

### 8.1.3. (2R-trans)-4-méthyl-2-(1H-tétrazol-4-yl)pipéridine

Dans un tube scellé, on place 0,7 g (3,1 mmoles) du composé précédemment obtenu en 8.1.2 et on ajoute 1,5 ml de diméthylformamide, 212 mg (3 mmoles) d'azoture de sodium et 180 mg (4 mmoles) de chlorure d'ammonium. On chauffe à 100 °C pendant 24 heures puis on évapore. On reprend le résidu dans un mélange de carbonate de sodium et d'éther diéthylique, on recueille la phase organique, on ajuste le pH à 2 avec de l'acide chlorhydrique 1 N et on extrait par de l'éther diéthylique. On récupère la phase organique, on la sèche sur du sulfate de magnésium et on évapore.
On obtient 0,3 g de produit sous forme d'une huile que l'on utilise telle quelle dans l'étape suivante.

### 8.1.4. trifluoroacétate de (2R-trans)-4-méthyl-2-(1H-tétrazol-4-yl)pipéridine

On dissout, sous azote, 242 mg (0,9 mmole) du composé précédemment obtenu en 8.1.3, dans 2 ml de dichlorométhane et on ajoute 2 ml d'acide trifluoroacétique. On agite pendant 1 heure à la température ambiante, on évapore et on reprend le résidu par du dichlorométhane. On évapore à nouveau.
On obtient 355 mg de produit sous forme d'une huile que l'on utilise telle quelle dans l'étape suivante.

### 8.2. chlorhydrate de [2R-[1(S), 2α, 4β]]-1-[5-(1H-imidazol-4-yl)-2-[[(3-méthyl-1,2,3,4-tétrahydroquinoléin-8-yl)sulfonyl]amino]-1-oxopentyl]-4-méthyl- 2-(1H-tétrazol-4-yl)pipéridine

### 8.2.1. [2R-[1(S), 2α, 4β]]-4-méthyl-1-[2-[[(3-méthyl-1,2,3,4-tétrahydroquinoléin-8-yl)sulfonyl]amino]-1-oxo-5-[1-(triphénylméthyl)-1H-imidazol-4- yl]pentyl]-2-(1H-tétrazol-4-yl)pipéridine

Sous azote, à 0 °C, on reprend 355 mg (0,9 mmole) du composé précédemment obtenu en 8.1, dans 6 ml de dichlorométhane. On ajoute 435 mg (0,98 mmole) d'hexafluorophosphate de [(benzotriazol-1-yl)oxy]tris(diméthylamino)phosphonium et 0,7 ml (5,64 mmoles) de N,N-diisopropyléthylamine et 570 mg du composé obtenu précédemment en 6.2. On laisse pendant une nuit à la température ambiante puis on verse le milieu réactionnel sur 50 ml d'acétate d'éthyle. On lave successivement par 50 ml d'acide chlorhydrique 0,1 N, 50 ml d'eau et finalement par 50 ml d'une solution saturée en chlorure de sodium. On sèche sur du sulfate de magnésium, on évapore, on reprend le résidu par 3 ml de dichlorométhane dont on a ajusté le pH à 5 avec de l'ammoniaque et on le purifie par chromatographie sur colonne de gel de silice en éluant par un mélange dichlorométhane/méthanol (97:3).
On obtient 450 mg de produit.
Point de fusion = 120-130 °C Rendement = 63 %

### 8.2.2. chlorhydrate de [2R-[1(S), 2α, 4β]]-1-[5-(1H-imidazol-4-yl)-2-[[(3-méthyl-1,2,3,4-tétrahydroquinoléin-8-yl)sulfonyl]amino]-1-oxopentyl]-4-méthyl- 2-(1H-tétrazol-4-yl)pipéridine

On chauffe au reflux sous azote, pendant 45 minutes, 450 mg (0,57 mmole) du composé précédemment obtenu dans un mélange contenant 5 ml d'acide acétique, 1 ml d'eau et 4 ml de tétrahydrofurane. On évapore et on purifie le résidu par chromatographie sur colonne de gel de silice en éluant par un mélange dichlorométhane/méthanol (90/10 à 80/20). On obtient 200 mg de produit sous forme de base.
On prépare le chlorhydrate à partir de la base que l'on a purifiée par chromatographie sur colonne de gel de silice en éluant par un gradient eau-acide trifluoroacétique à 0,1 %/acétonitrile (95/5 à 5/95). On réunit les fractions intéressantes et on les évapore. On reprend le résidu par de l'acide chlorhydrique 0,1 N dans l'isopropanol et on évapore de nouveau. On reprend le résidu par de l'éther et on le triture.
On obtient 0,15 g de produit sous forme de chlorhydrate.
Point de fusion = 140-150 °C Rendement = 49 %
[α]$\frac{\text{20}}{\text{D}}$ = + 115 (c = 0,2; méthanol)

Le tableau suivant illustre les structures chimiques et les propriétés physiques de quelques composés selon l'invention.

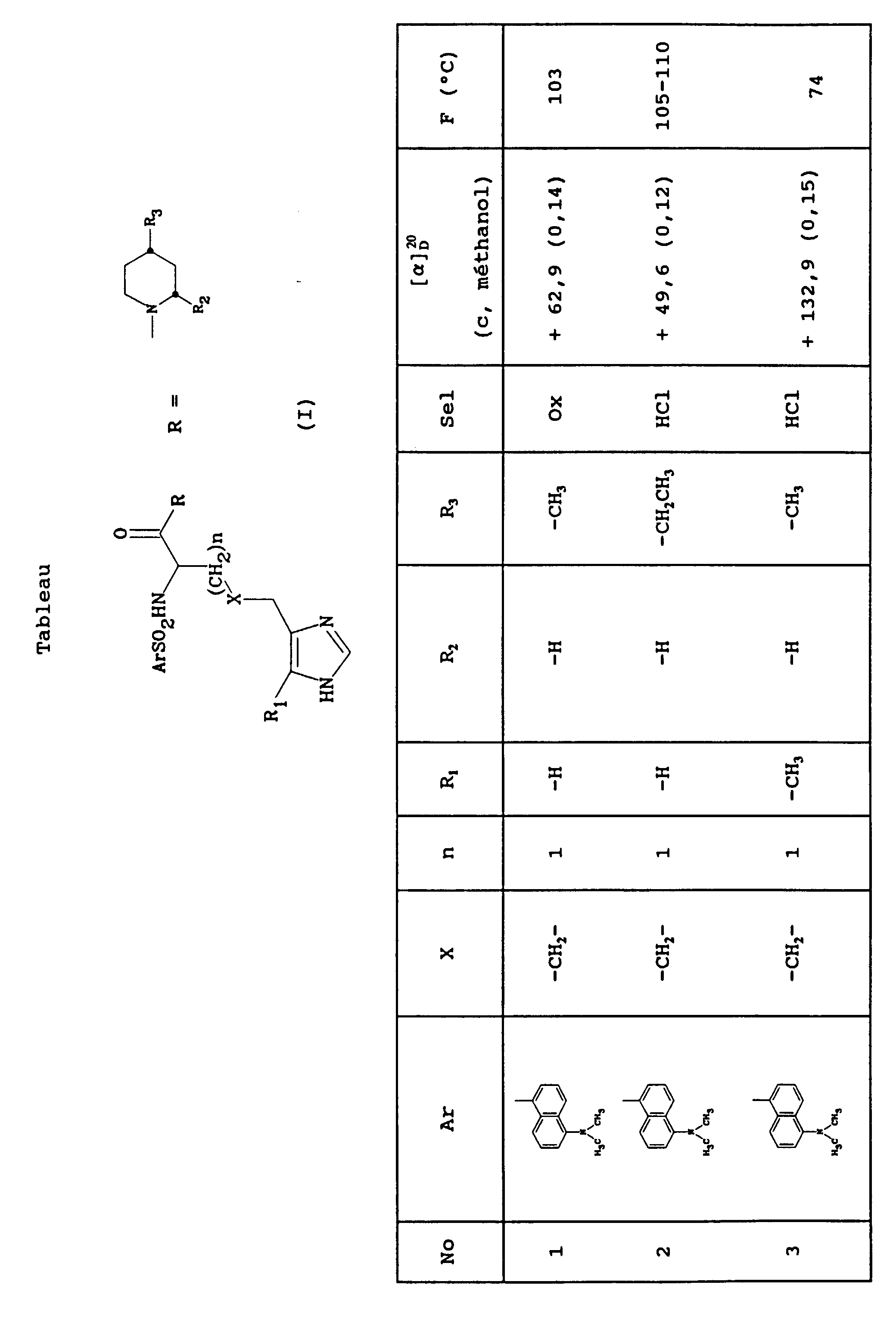

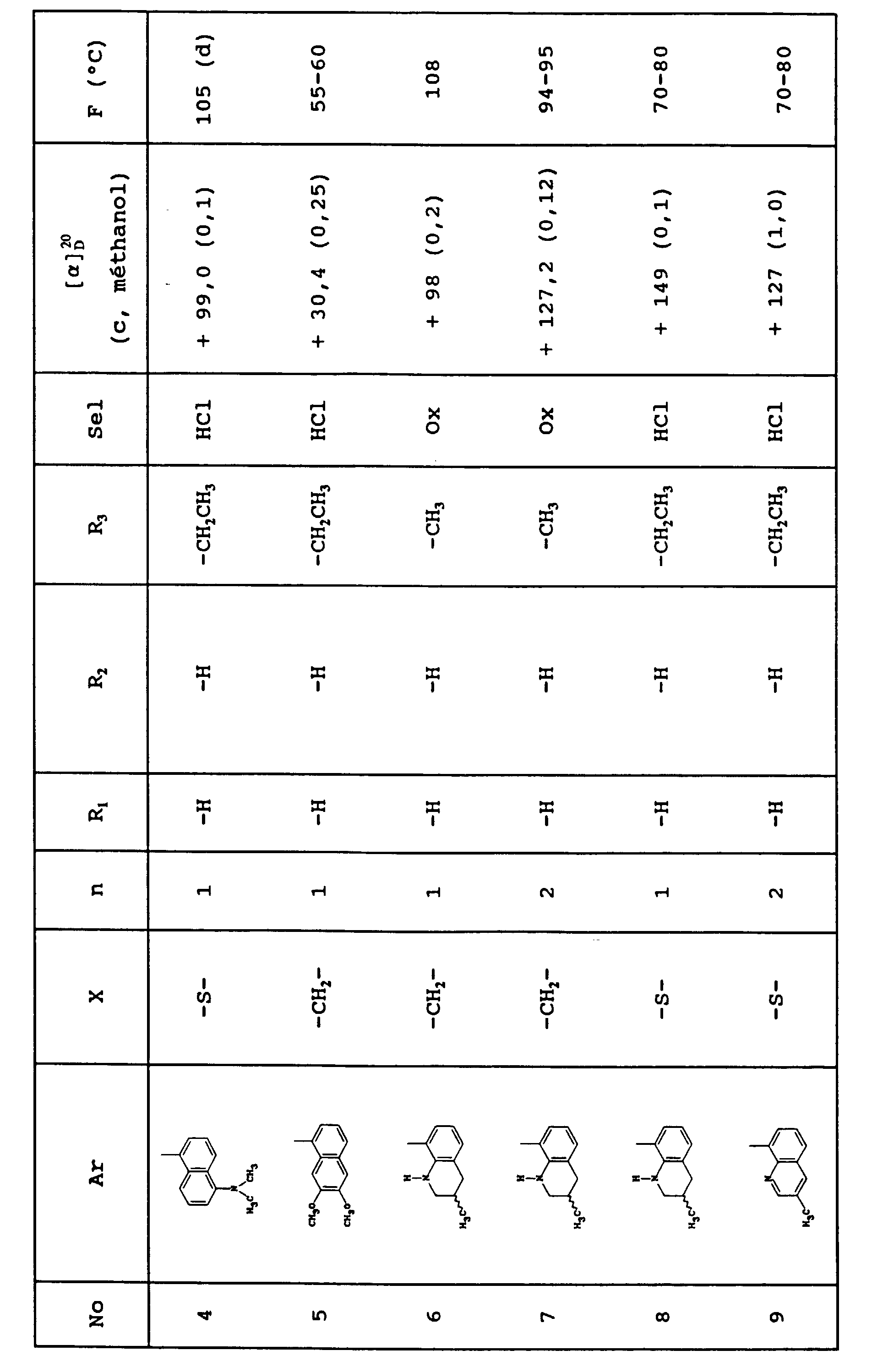

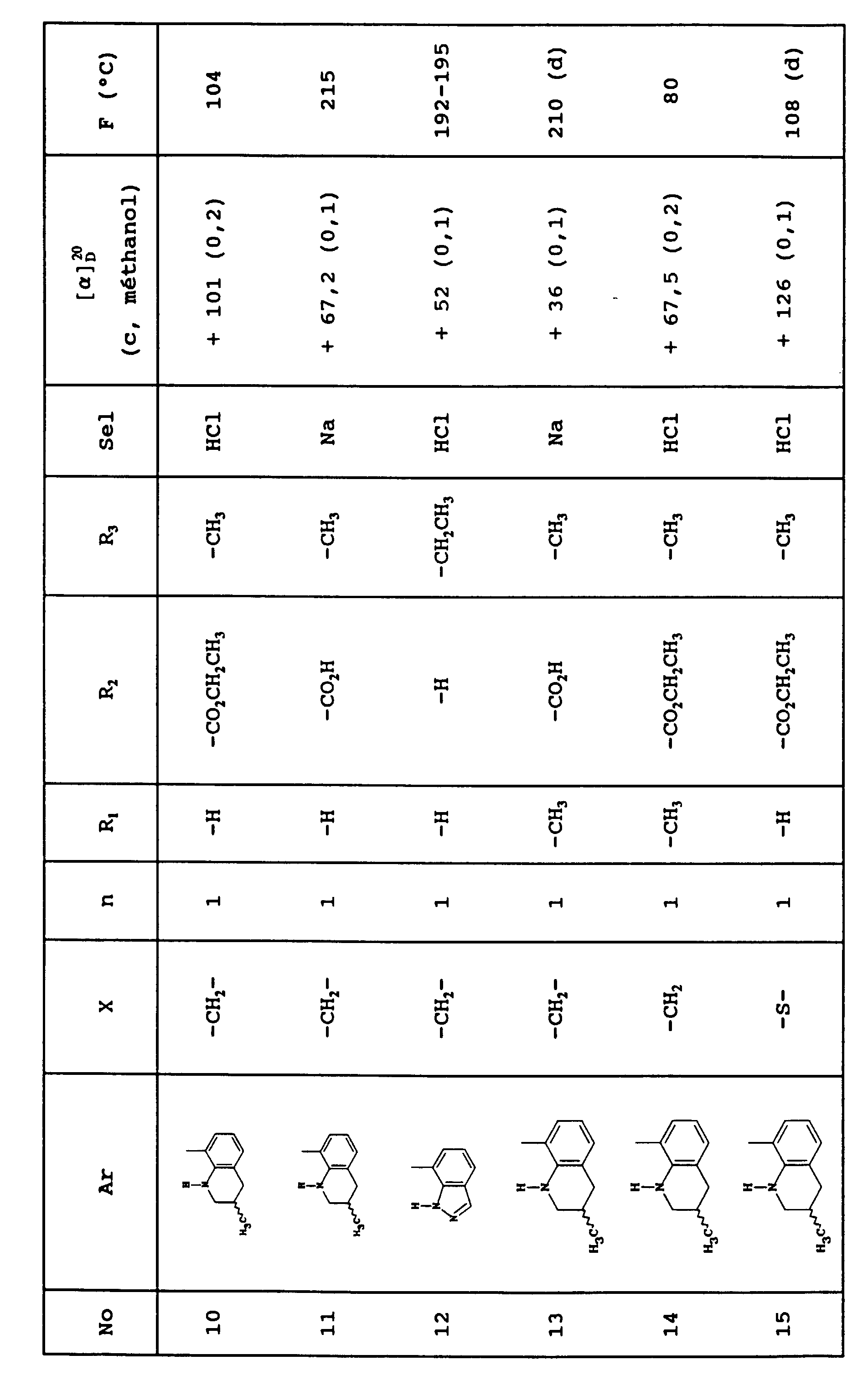

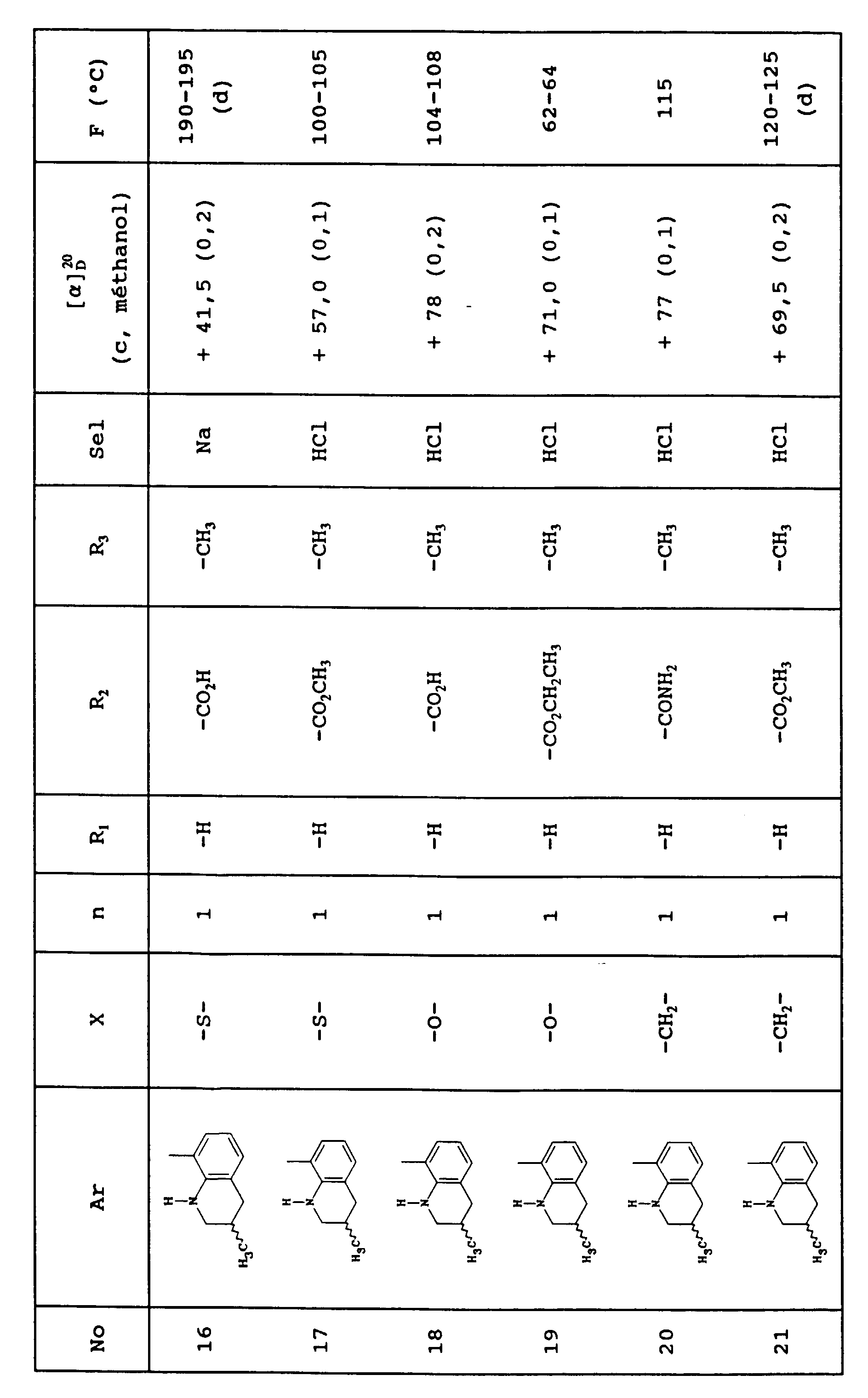

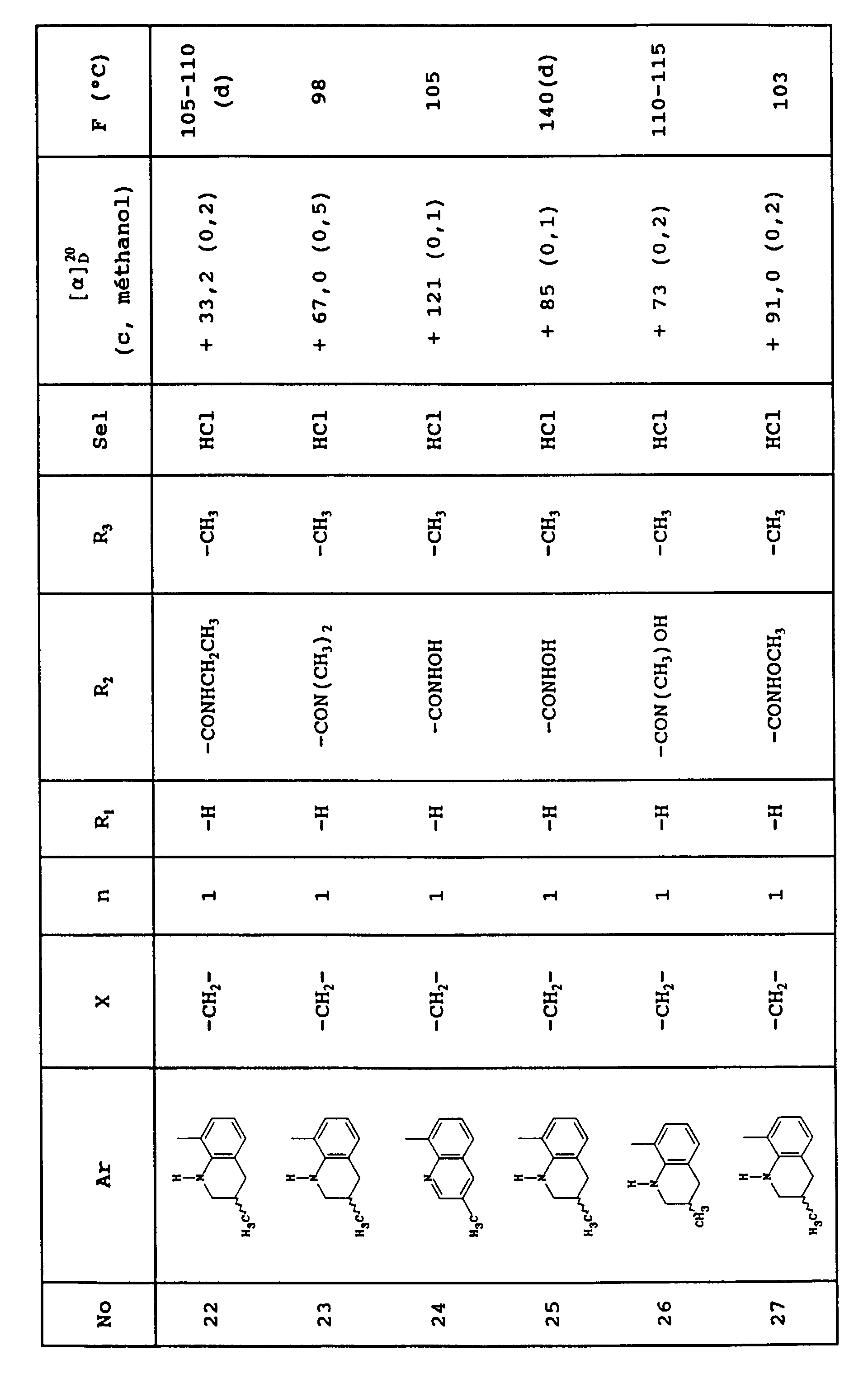

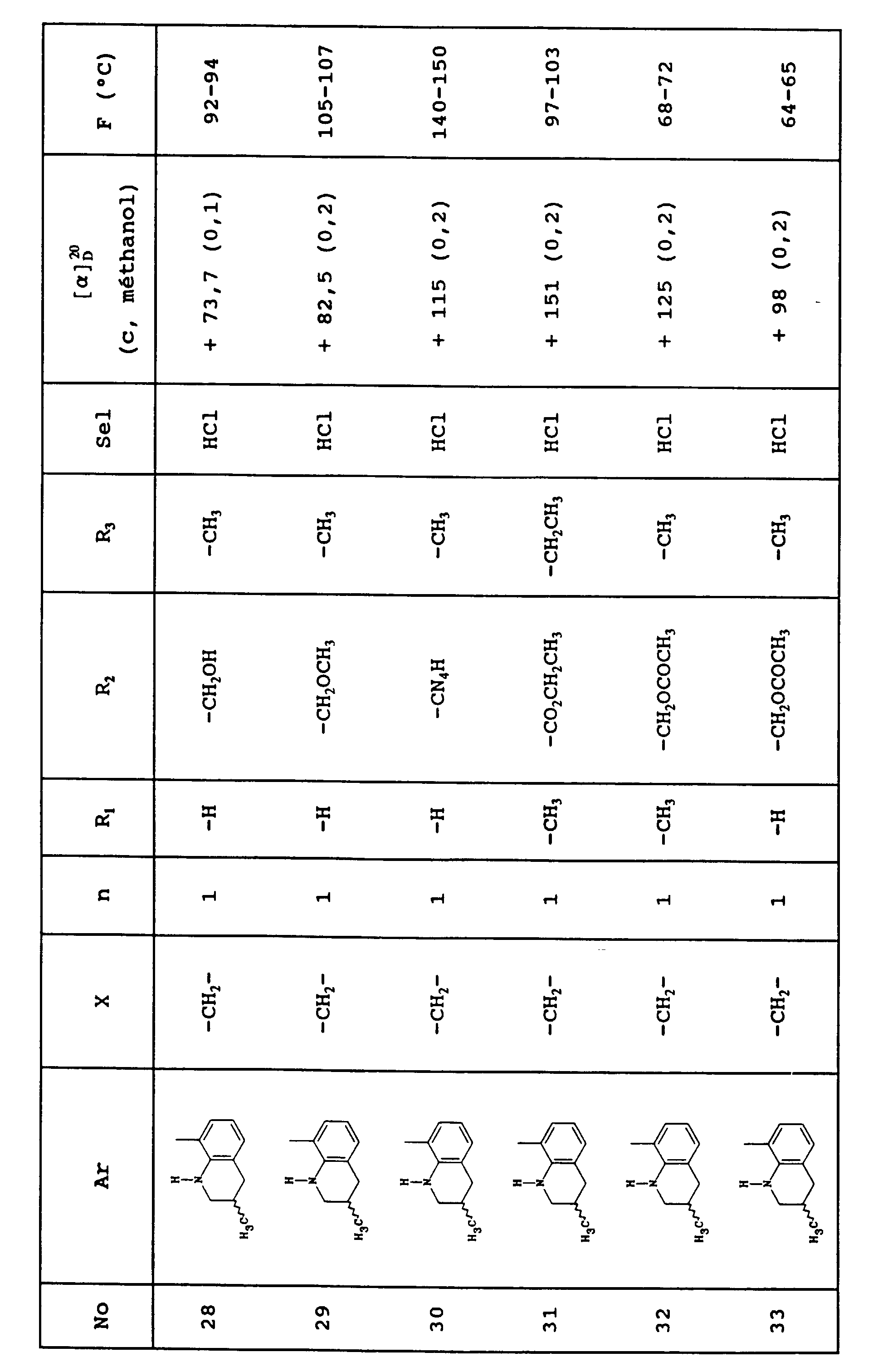

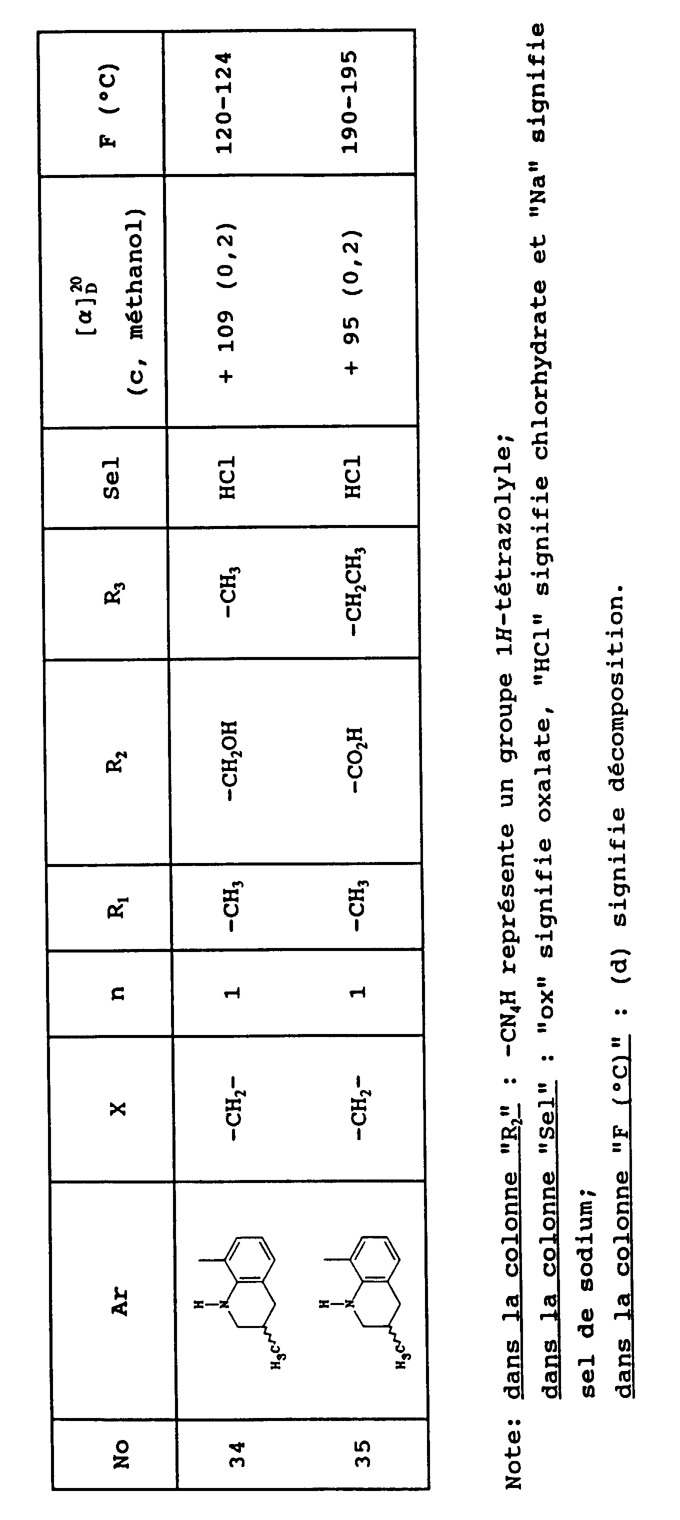

Les composés de l'invention ont été testés vis-à-vis de la thrombine et de la trypsine *in*-*vitro* dans les tests suivants.

### 1. Précipitation du fibrinogène humain par la thrombine bovine.

On incube le composé à tester ou son véhicule (10 µl), pendant 2 minutes, à 37°C, dans une solution de fibrinogène humain (200 µl, 2 mg/ml dans du sérum physiologique). Ensuite, on ajoute 200 µl de thrombine bovine dissous dans de l'eau distillée. La concentration'finale de la thrombine est de 0,5 unités NIH/ml. On agite et on note le temps de formation, exprimé en secondes, d'un réseau de fibrine visible. L'inhibition de la fibrinoformation est quantifiée par le calcul de la concentration de composé qui augmente le temps de précipitation de 100 % (CA₁₀₀). Les résultats sont exprimés en CA₁₀₀, moyenne ± s.e.m. d'au moins trois expériences.

### 2. Précipitation du fibrinogène humain par la thrombine humaine.

On incube le composé à tester ou son véhicule (10 µl), pendant 2 minutes, à 37°C, dans une solution de fibrinogène humain (200 µl, 2 mg/ml dans du sérum physiologique). Ensuite, on ajoute 200 µl de thrombine humaine dissous dans de l'eau distillée. La concentration finale de la thrombine est de 2 unités NIH/ml. On agite et on note le temps de formation, exprimé en secondes, d'un réseau de fibrine visible. L'inhibition de la fibrinoformation est quantifiée par le calcul de la concentration de composé qui augmente le temps de précipitation de 100 % (CA₁₀₀). Les résultats sont exprimés en CA₁₀₀, moyenne ± s.e.m. d'au moins trois expériences.

### 3. Coagulation du plasma de rat par la thrombine bovine.

On anesthésie des rats CD mâles, pesant 150 à 200 g, avec du nembutal (60 mg/kg, 0,1 ml/kg). On prélève le sang sur du citrate trisodique à 3,8 % (1 vol/9 vol de sang) à partir du sinus rétro-orbital. On prépare le plasma par centrifugation à 3600 g pendant 15 minutes, à la température ambiante. On incube le composé à tester ou son véhicule (10 µl) avec 200 µl de plasma, à 37°C, pendant 2 minutes, avant l'addition de 200 µl d'une solution de thrombine bovine. La concentration finale en thrombine est de 0,75 unités NIH/ml. On note le temps de coagulation, exprimé en secondes.
L'inhibition de la thrombine est quantifiée par le calcul de la concentration qui augmente le temps de coagulation de 100% (CA ₁₀₀).

### 4. Agrégation des plaquettes de lapin induite par la thrombine humaine.

On prélève le sang par ponction cardiaque sur du citrate trisodique à 3,8 % (1 vol/9 vol de sang). On le centrifuge à 250 g pendant 10 minutes. On prélève le plasma riche en plaquettes (PRP) ainsi obtenu et on réalise la numération des plaquettes.
Au PRP, on ajoute 2 ng/ml de prostacycline en solution dans du tampon tris à pH 9,0 glacé. On centrifuge à 110 g, pendant 10 minutes et on décante. On ajoute à nouveau de la prostacycline, en solution dans de l'hydroxyde de sodium 50 mM à pH 12, de manière à avoir une concentration finale de 200 ng/ml. On centrifuge à nouveau le PRP à 800 g pendant 10 minutes. On élimine le plasma pauvre en plaquettes et on met le culot en suspension dans un volume de tyrode contenant 200 ng/ml de prostacycline, volume égal au volume initial de PRP. On centrifuge cette suspension à 800 g pendant 10 minutes. On recommence une seconde fois et dans les mêmes conditions, la mise en suspension du culot et la centrifugation. On remet le culot final en suspension dans une solution de tyrode sans prostacycline et on laisse au repos pendant 2 heures pour permettre l'élimination complète de la prostacycline. On induit l'agrégation de ces plaquettes avec de la thrombine humaine à la concentration finale de 0,3 unités NIH/ml. On enregistre les variations de densité optique au moyen d'un agrégomètre à 4 canaux. On ajoute le composé à tester ou son véhicule à la suspension de plaquettes (volume maximal ajouté de 3 µl), 2 minutes avant l'addition de thrombine. On détermine la concentration qui inhibe l'agrégation de 50 % (CI₅₀). Les résultats sont exprimés en CI₅₀, moyenne ± s.e.m. d'au moins trois expériences.

### 5. Activité vis-à-vis de la trypsine bovine

On incube le composé à tester ou son véhicule (50 µl), pendant 5 minutes, à la température ambiante, avec 50 µl de trypsine bovine dissous dans du tampon tris HCl à pH 8,0. La concentration finale en trypsine est de 229 unités/ml. On déclenche la réaction par l'addition du substrat, la *N* α-benzoyl-L-arginine-4 nitro-aniline (50 µl, concentration finale 50 µM). On incube pendant 20 minutes à la température ambiante et on mesure la densité optique de la 4-nitro-aniline libérée, à 405 nm. On calcule la concentration de 4-nitro-aniline à partir d'une courbe d'étalonnage, après avoir soustrait la densité optique des "blancs" (100 µl de tampon + 50 µl de substrat). On détermine la concentration qui inhibe de 50 % l'activité enzymatique (CI₅₀). Les résultats sont exprimés en CI₅₀, moyenne ± s.e.m..

Parallèlement les composés de l'invention ont été testés vis-à--vis de la coagulation du plasma de rat *ex-vivo*. On traite les animaux avec le composé à tester ou avec son véhicule, par voie i.v. ou par voie orale, avant de prélever le sang. On mesure le temps de thrombine comme décrit en 3.

Les composés de l'invention sont des inhibiteurs de la thrombine avec des CA₁₀₀ et des CI₅₀ comprises entre 10⁻⁹ et 10⁻⁶ M. Ils ne présentent pas ou présentent peu d'activité inhibitrice vis-à-vis de la trypsine bovine, ce qui démontre leur spécificité.
Ils inhibent la coagulation du plasma de rat à des doses inférieures ou égales à 1 mg/kg i.v. et sont également actifs par voie orale.

Les composés de l'invention peuvent être utiles dans toutes les indications cliniques liées à la thrombose ou dans celles où des complications thrombotiques pourraient intervenir.

A cet effet ils peuvent être présentés sous toutes formes appropriées à l'administration orale, parentérale ou intraveineuse, telles que comprimés, dragées, gélules, capsules, suspensions ou solutions buvables ou injectables, etc. en association avec des excipients convenables, et dosées pour permettre une administration de 1 à 1000 mg par jour et par patient, en une ou plusieurs doses.

## Revendications

1. Composés répondant à la formule (I) 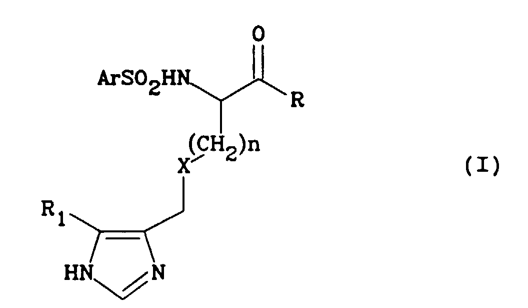 dans laquelle
R représente un groupe 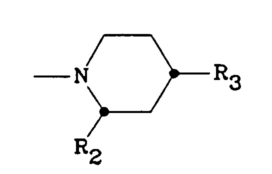 où R₂ est un atome d'hydrogène, un groupe (C₁-C₄)alcoxycarbonyle, un groupe carboxylique, un groupe carboxylate de sodium, un groupe -CH₂OR₄, R₄ étant un atome d'hydrogène, un groupe (C₁-C₄)alkyle ou un groupe (C₁-C₄)acyle, un groupe amide de formule -CONR₅R₆ ou un groupe -CN₄R₅, R₅ étant un atome d'hydrogène ou un groupe (C₁-C₄)alkyle et R₆ étant un atome d'hydrogène, un groupe (C₁-C₄)alkyle, un groupe hydroxy, un groupe (C₁-C₄)alcoxy ou un groupe (C₁-C₃)alcoxyphényle et R₃ est un groupe (C₁-C₄)alkyle,
R₁ représente soit un atome d'hydrogène, soit un groupe (C₁-C₄)alkyle,
X représente soit un atome de soufre, soit un atome d'oxygène, soit un groupe méthylène,
n = 1 ou 2 et
Ar représente soit un groupe naphtalén-1-yle substitué par un groupe di(C₁-C₄)alkylamino, soit un groupe 6,7-di(C₁-C₄)alcoxy naphtalén-1-yle, soit un groupe quinoléin-8-yle substitué en 3 par un groupe (C₁-C₄)alkyle, soit un groupe 1,2,3,4-tétrahydroquinoléin-8-yle substitué en 3 par un groupe (C₁-C₄)alkyle, soit un groupe 1*H*-indazol-7-yle
ainsi que leurs sels organiques ou inorganiques pharmaceutiquement acceptables.

2. Composés selon la revendication 1 caractérisés en ce que
R représente un groupe 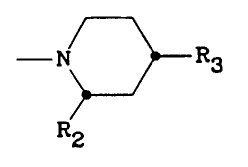 où R₂ est un atome d'hydrogène, un groupe (C₁-C₄)alcoxycarbonyle, un groupe carboxylique, un groupe carboxylate de sodium, un groupe -CH₂OR₄, R₄ étant un atome d'hydrogène, un groupe (C₁-C₄)alkyle ou un groupe (C₁-C₄)acyle, un groupe amide de formule -CONR₅R₆ ou un groupe -CN₄R₅, R₅ étant un atome d'hydrogène ou un groupe (C₁-C₄)alkyle et R₆ étant un atome d'hydrogène, un groupe (C₁-C₄)alkyle, un groupe hydroxy, un groupe (C₁-C₄)alcoxy ou un groupe (C₁-C₃)alcoxyphényle et R₃ est un groupe (C₁-C₄)alkyle,
R₁ représente soit un atome d'hydrogène, soit un groupe (C₁-C₄)alkyle,
X représente soit un atome de soufre, soit un atome d'oxygène, soit un groupe méthylène,
n = 1 ou 2 et
Ar représente soit le groupe 5-(diméthylamino)-naphtalén-1-yle, soit le groupe 6,7-diméthoxy-naphtalén-1-yle, soit le groupe 3-méthyl-quinoléin-8-yle, soit le groupe 3-méthyl-1,2,3,4-tétrahydroquinoléin-8-yle et qui peuvent éventuellement exister sous forme de diastéréoisomères.

3. Composés selon la revendication 2 caractérisés en ce que
R représente un groupe 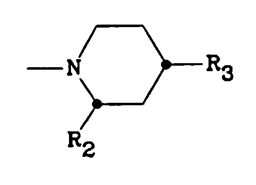 où R₂ est un groupe carboxylique, un groupe éthoxycarbonyle, un groupe hydroxycarboxamide, un groupe hydroxyméthyle, ou un groupe 1*H*-tétrazolyle et R₃ est un groupe méthyle ou un groupe éthyle,
R₁ est un atome d'hydrogène ou un groupe méthyle,
X est un groupe méthylène
n = 1 et
Ar est le radical 3-méthyl-1,2,3,4-tétrahydroquinoléin-8-yle.

4. Composés selon l'une quelconque des revendications 1 à 3 caractérisés en ce que la configuration du groupe pipéridinyle est [2R, 4R] et la configuration du carbone asymétrique de la partie aminée centrale 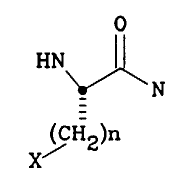 est [S].

5. Procédé de préparation des composés selon la revendication 1, de formule générale (I bis) 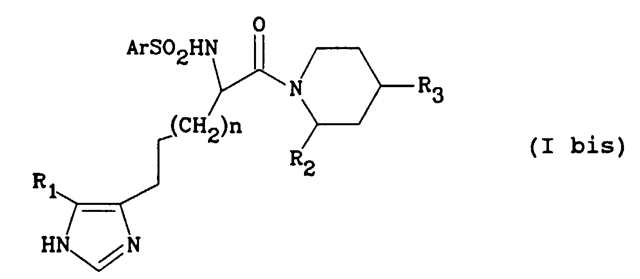 dans laquelle n = 1 ou 2 et R₁, R₂, R₃ et Ar sont tels que définis dans la revendication 1, procédé caractérisé en ce que l'on fait réagir un alcool de formule (II) 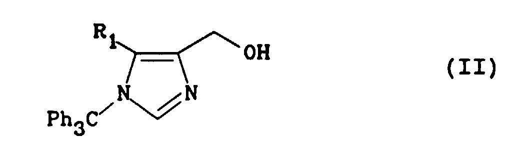 dans laquelle R₁ est tel que défini dans la revendication 1, avec du chlorure de thionyle,
puis on fait réagir le composé obtenu avec de la triphénylphosphine pour obtenir un composé de formule (III) 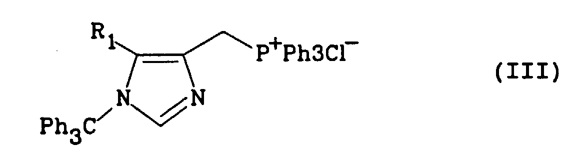 que l'on fait réagir avec un composé de formule (IV) 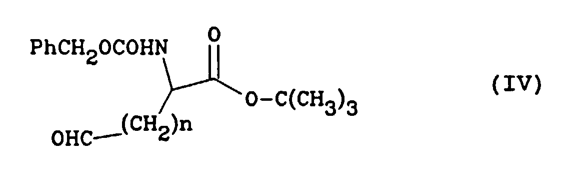 pour obtenir un composé de formule (V) 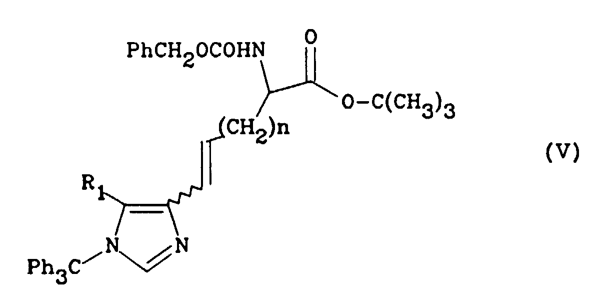 que l'on soumet à une hydrogénation catalytique pour obtenir un composé de formule (VI) 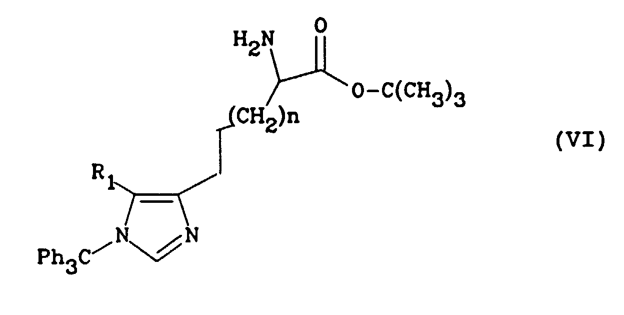 que l'on fait réagir avec un chlorure d'arylsulfonyle de formule
ArSO₂Cl
dans laquelle Ar est tel que défini dans la revendication 1, pour obtenir un composé de formule (VII) 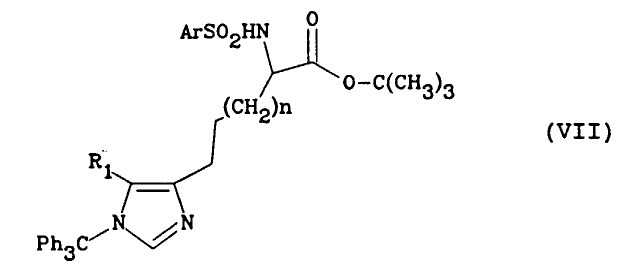 dont on réalise la déprotection pour obtenir un composé de formule (VIII) 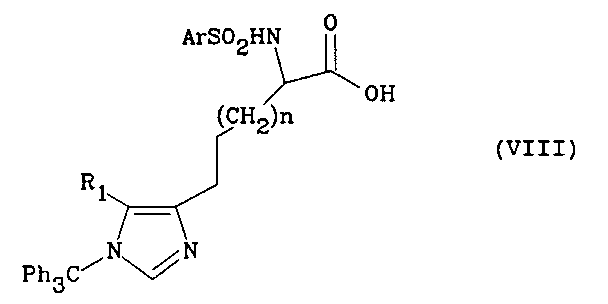 que l'on condense avec un composé de formule (IX) 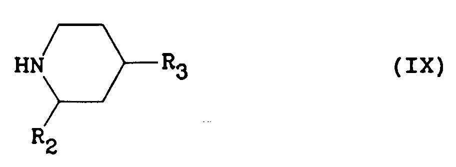 dans laquelle R₂ et R₃ sont tels que définis dans la revendication 1,
pour obtenir un composé de formule (X) 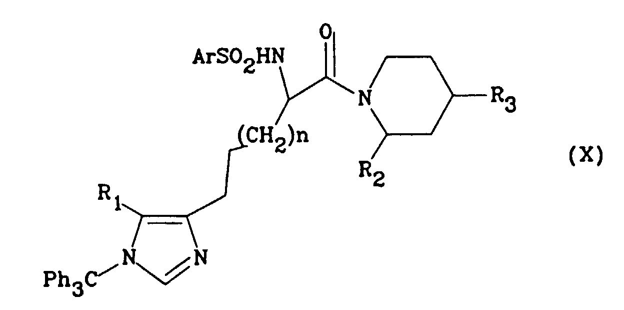 que l'on soumet à une réaction de déprotection pour obtenir un composé de formule (I bis).

6. Procédé de préparation des composés selon la revendication 1, de formule (I ter) 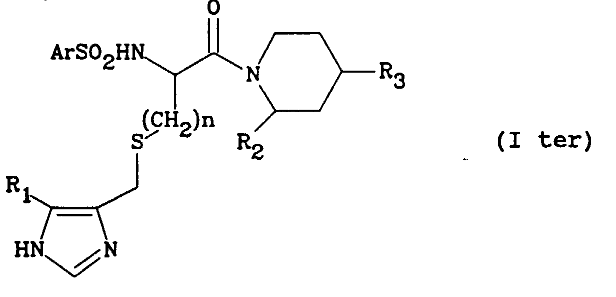 dans laquelle n = 1 et R₁, R₂, R₃ et Ar sont tels que définis dans la revendication 1, procédé caractérisé en ce que l'on fait réagir un alcool de formule (II) 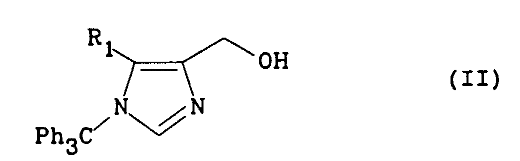 dans laquelle R₁ est tel que défini dans la revendication 1 avec du chlorure de thionyle,
puis on fait réagir le composé obtenu avec de la *L*-cystéine pour obtenir un composé de formule (XII) 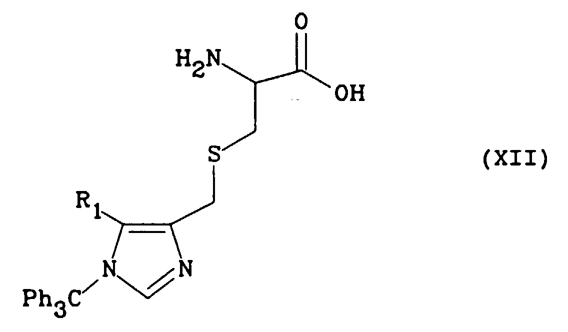 que l'on fait réagir avec un chlorure d'arylsulfonyle de formule
ArSO₂Cl
dans laquelle Ar est tel que défini dans la revendication 1 pour obtenir un composé de formule (XIII) 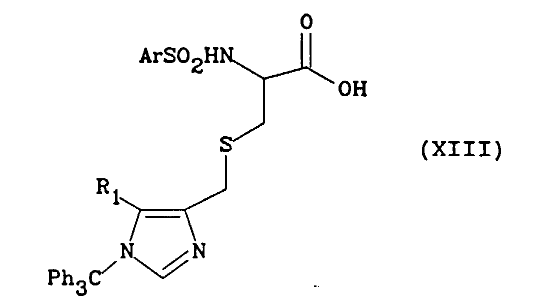 que l'on condense avec un composé de formule (IX) 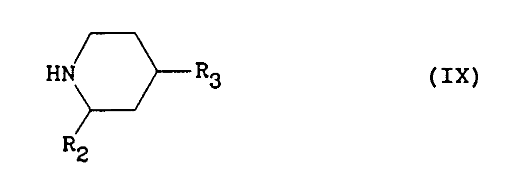 dans laquelle R₂ et R₃ sont tels que définis dans la revendication 1,
pour obtenir un composé de formule (XIV) 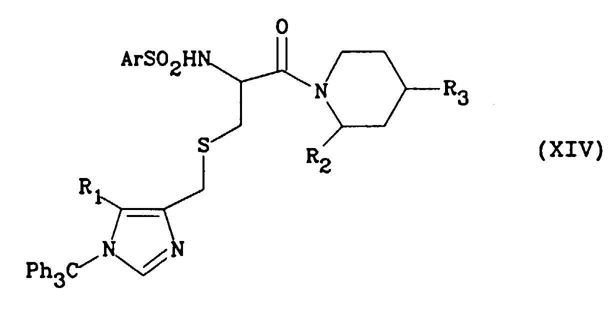 que l'on déprotège pour obtenir un composé de formule (I ter) dans laquelle n = 1.

7. Procédé de préparation des composés selon la revendication 1, de formule (I ter) 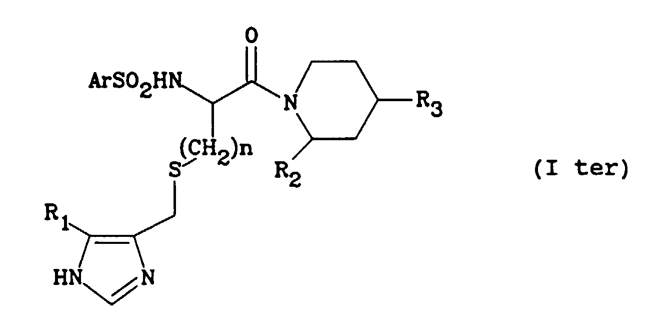 dans laquelle n = 2 et R₁, R₂, R₃ et Ar sont tels que définis dans la revendication 1, procédé caractérisé en ce que l'on fait réagir un alcool de formule (II) 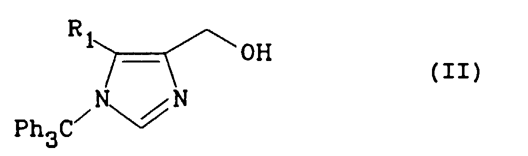 dans laquelle R₁ est tel que défini dans la revendication 1, avec du chlorure de thionyle,
puis on fait réagir le composé obtenu avec du thioacétate de potassium pour obtenir un composé de formule (XV) 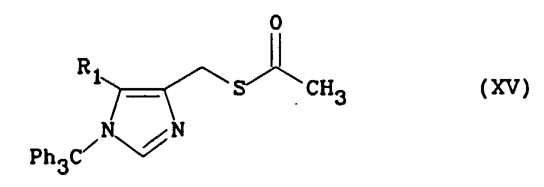 que l'on fait réagir avec un tosylate de formule (XVI) 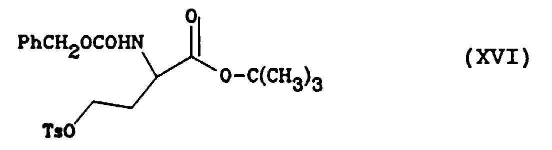 pour obtenir un composé de formule (XVII) 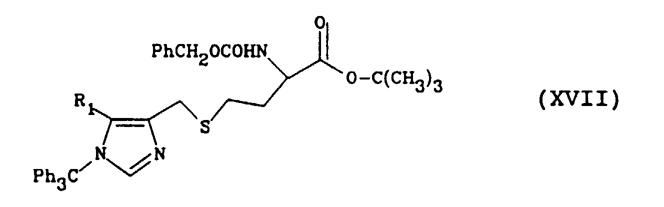 que l'on déprotège pour obtenir un composé de formule (XVIII) 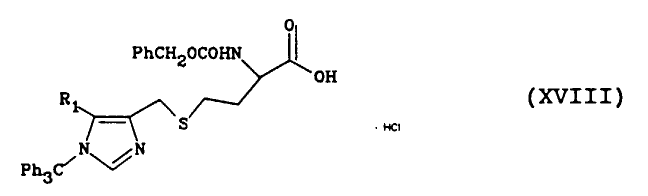 que l'on condense avec un composé de formule (IX) 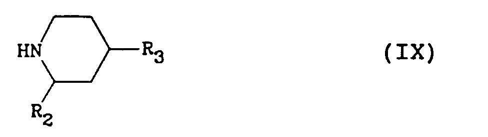 dans laquelle R₂ et R₃ sont tels que définis dans la revendication 1,
pour obtenir un composé de formule (XIX) 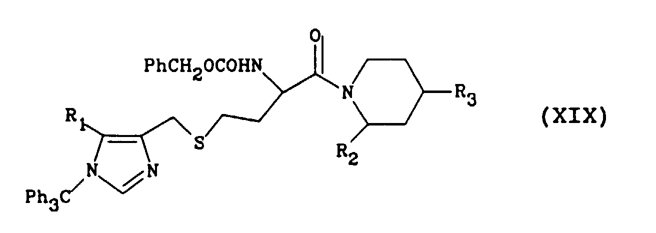 que l'on déprotège pour obtenir un composé de formule (XX) 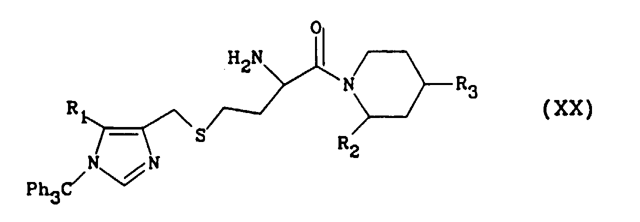 que l'on fait réagir avec un chlorure d'arylsulfonyle de formule
ArSO₂Cl
dans laquelle Ar est tel que défini dans la revendication 1, pour obtenir un composé de formule (XXI) 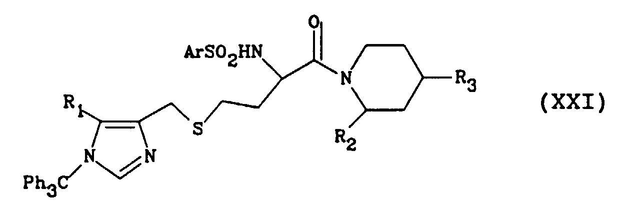 que l'on déprotège pour obtenir un composé de formule (I ter) dans laquelle n = 2.

8. Procédé de préparation des composés selon la revendication 1, de formule (I quater) 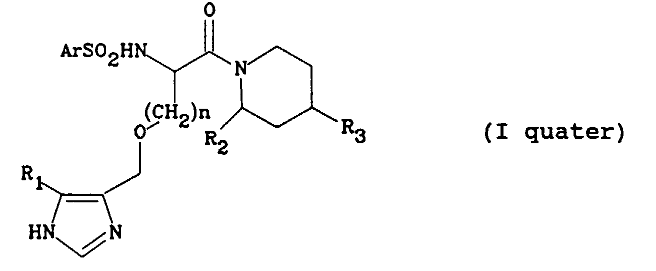 dans laquelle n= 1 ou 2 et R₁, R₂, R₃ et Ar sont tels que définis dans la revendication 1, procédé caractérisé en ce que l'on fait réagir un alcool de formule (II) 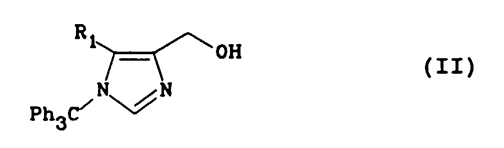 dans laquelle R₁ est tel que défini dans la revendication 1, avec du chlorure de thionyle,
puis on fait réagir le composé obtenu avec un composé de formule (XXII) préalablement traité par de l'hydrure de sodium 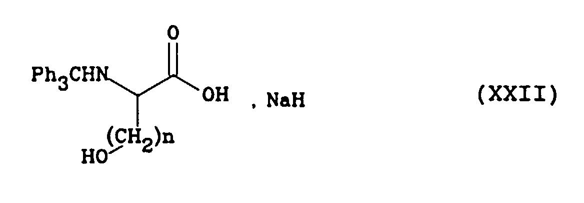 pour obtenir un composé de formule (XXIII) 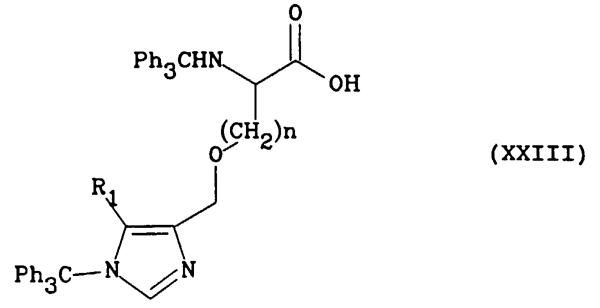 que l'on condense avec un composé de formule (IX) 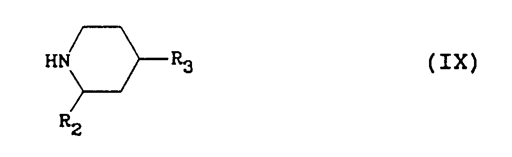 dans laquelle R₂ et R₃ sont tels que définis dans la revendication 1,
pour obtenir un composé de formule (XXIV) 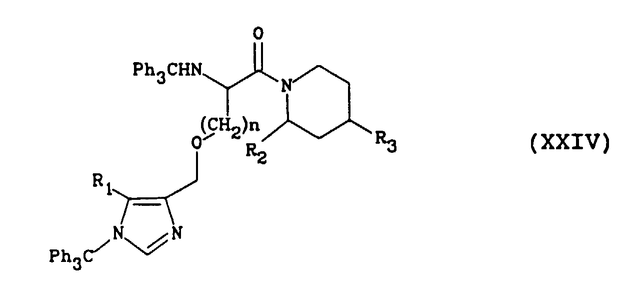 que l'on déprotège pour obtenir un composé sur lequel on fait réagir un chlorure d'arylsulfonyle de formule
ArSO₂Cl
dans laquelle Ar est tel que défini dans la revendication 1, et enfin on déprotège le noyau imidazolé pour obtenir un composé de formule (I quater).

9. Médicament, caractérisé en ce qu'il contient un composé selon l'une quelconque des revendications 1 à 4.

10. Composition pharmaceutique, caractérisée en ce qu'elle contient un composé selon l'une quelconque des revendications 1 à 4, en association avec tout excipient approprié.

## Patentansprüche

1. Verbindungen der Formel (I) 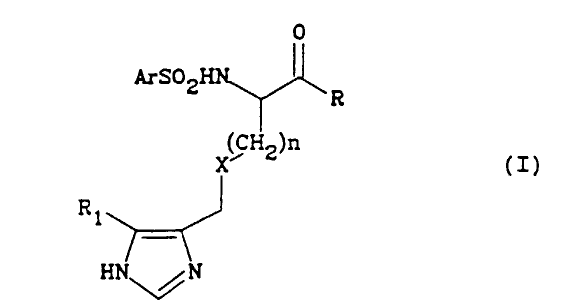 in der
R eine Gruppe 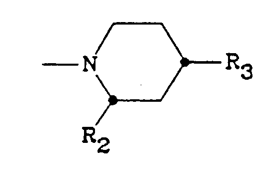 in der R₂ ein Wasserstoffatom, eine (C₁-C₄)-Alkoxycarbonylgruppe, eine Carboxylgruppe, eine Natriumcarboxylatgruppe, eine Gruppe -CH₂OR₄, worin R₄ für ein Wasserstoffatom, eine (C₁-C₄)-Alkylgruppe oder eine (C₁-C₄)-Acylgruppe steht, eine Amidgruppe der Formel -CONR₅R₆ oder eine Gruppe -CN₄R₅, worin R₅ für ein Wasserstoffatom oder eine (C₁-C₄)-Alkylgruppe und R₆ für ein Wasserstoffatom, eine (C₁-C₄)-Alkylgruppe, eine Hydroxygruppe, eine (C₁-C₄)-Alkoxygruppe oder eine (C₁-C₃)-Alkoxyphenylgruppe stehen, und R₃ eine (C₁-C₄)-Alkylgruppe darstellen,
R₁ entweder ein Wasserstoffatom oder eine (C₁-C₄)-Alkylgruppe,
X entweder ein Schwefelatom oder ein Sauerstoffatom oder eine Methylengruppe,
n = 1 oder 2 und
Ar entweder eine Naphthalin-1-yl-gruppe, die durch eine Di-(C₁-C₄)-alkylaminogruppe substituiert ist, oder eine 6,7-Di-(C₁-C₄)-alkoxy-naphthalin-1-yl-gruppe oder eine Chinolin-8-yl-gruppe, die in der 3-Stellung durch eine (C₁-C₄)-Alkylgruppe substituiert ist, oder eine 1,2,3,4-Tetrahydrochinolin-8-yl-gruppe, die in der 3-Stellung durch eine (C₁-C₄)-Alkylgruppe substituiert ist, oder eine 1H-Indazol-7-yl-gruppe bedeuten,
sowie deren pharmazeutisch annehmbare organische oder anorganische Salze.

2. Verbindungen nach Anspruch 1, dadurch gekennzeichnet, daß R eine Gruppe der Formel 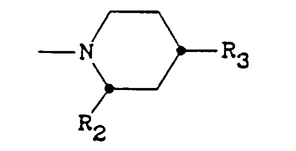 in der
R₂ ein Wasserstoffatom, eine (C₁-C₄)-Alkoxycarbonylgruppe, eine Carboxylgruppe, eine Natriumcarboxylatgruppe, eine Gruppe -CH₂OR₄, worin R₄ für ein Wasserstoffatom, eine (C₁-C₄)-Alkylgruppe oder eine (C₁-C₄)-Acylgruppe steht, eine Amidgruppe der Formel -CONR₅R₆ oder eine Gruppe -CN₄R₅, worin R₅ für ein Wasserstoffatom oder eine (C₁-C₄)-Alkylgruppe und R₆ für ein Wasserstoffatom, eine (C₁-C₄)-Alkylgruppe, eine Hydroxygruppe, eine (C₁-C₄)-Alkoxygruppe oder eine (C₁-C₃)-Alkoxyphenylgruppe stehen, und R₃ eine (C₁-C₄)-Alkylgruppe darstellen.
R₁ entweder ein Wasserstoffatom oder eine (C₁-C₄)-Alkylgruppe,
X entweder ein Schwefelatom oder ein Sauerstoffatom oder eine Methylengruppe, n = 1 oder 2 und
Ar entweder die 5-(Dimethylamino)-naphthalin-1-yl-gruppe oder die 6,7-Dimethoxy-naphthalin-1-yl-gruppe oder die 3-Methyl-chinolin-8-yl-gruppe oder die 3-Methyl-1,2,3,4-tetrahydrochinolin-8-yl-gruppe bedeuten,
die gegebenenfalls in Form der Diastereoisomeren vorliegen können.

3. Verbindungen nach Anspruch 2, dadurch gekennzeichnet, daß R eine Gruppe der Formel 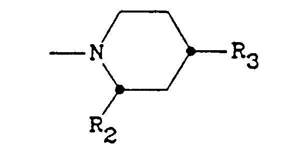 in der
R₂ eine Carboxylgruppe, eine Ethoxycarbonylgruppe, eine Hydroxycarboxamidgruppe, eine Hydroxymethylgruppe oder eine 1H-Tetrazolylgruppe und
R₃ eine Methylgruppe oder eine Ethylgruppe darstellen.
R₁ ein Wasserstoffatom oder eine Methylgruppe,
X eine Methylengruppe,
n = 1 und
Ar die 3-Methyl-1,2,3,4-tetrahydrochinolin-8-yl-gruppe bedeuten.

4. Verbindungen nach irgendeinem der Ansprüche 1 bis 3, dadurch gekennzeichnet, daß die Konfiguration der Piperidinylgruppe [2R, 4R] ist und die Konfiguration des asymmetrischen Kohlenstoffatoms des zentralen Aminorests 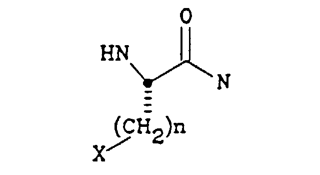 die [S]-Konfiguration ist.

5. Verfahren zur Herstellung der Verbindungen nach Anspruch 1 der allgemeinen Formel (I bis) 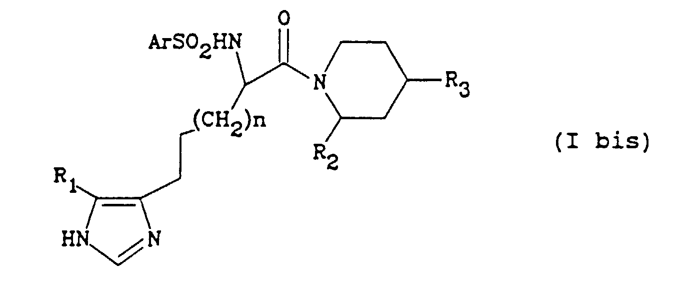 in der n = 1 oder 2 bedeutet und R₁, R₂, R₃ und Ar die in Anspruch 1 angegebenen Bedeutungen besitzen, dadurch gekennzeichnet, daß man einenAlkohol der Formel (II) 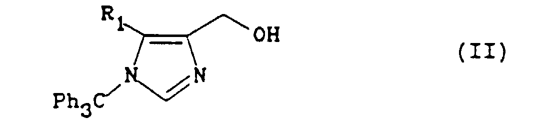 in der R₁ die in Anspruch 1 angegebenen Bedeutungen besitzt, mit Thionylchlorid umsetzt,
dann die erhaltene Verbindung mit Triphenylphosphin umsetzt zur Bildung einer Verbindung der Formel (III) 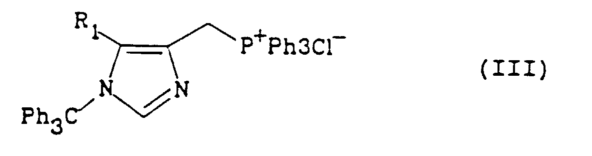 welche man mit einer Verbindung der Formel (IV) umsetzt 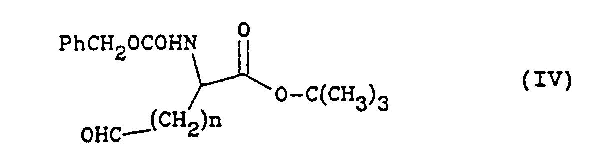 zur Bildung einer Verbindung der Formel (V) 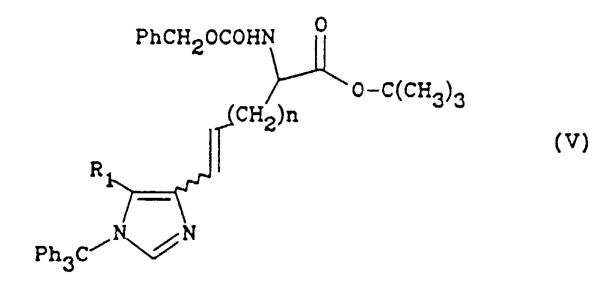 welche man einer katalytischen Hydrierung unterwirft zur Bildung einer Verbindung der Formel (VI) 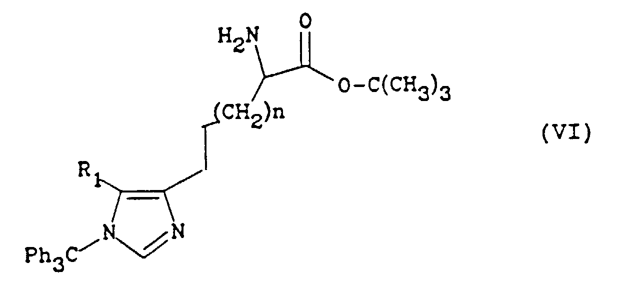 die man mit einem Arylsulfonylchlorid der Formel
ArSO₂Cl
umsetzt, in der Ar die in Anspruch 1 angegebenen Bedeutungen besitzt, zur Bildung einer Verbindung der Formel (VII) 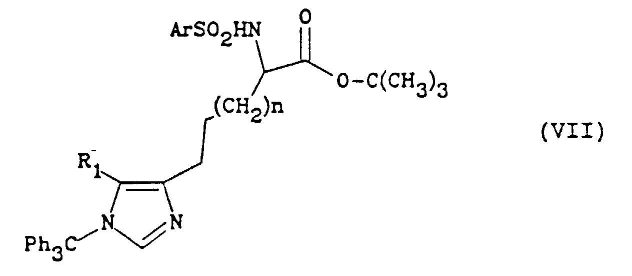 von welcher man die Schutzgruppe abspaltet zur Bildung einer Verbindung der Formel (VIII) 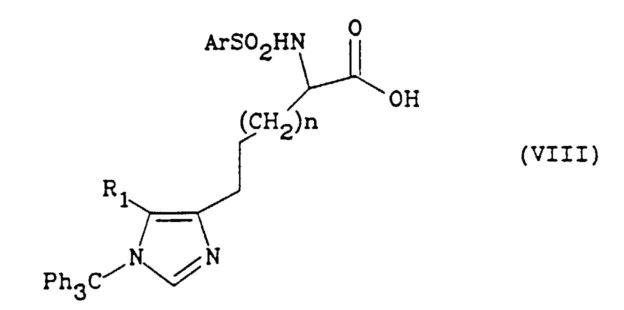 welche man mit einer Verbindung der Formel (IX) 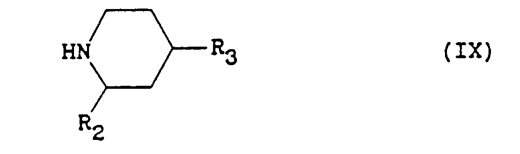 in der R₂ und R₃ die in Anspruch 1 angegebenen Bedeutungen besitzen, kondensiert
zur Bildung einer Verbindung der Formel (X) 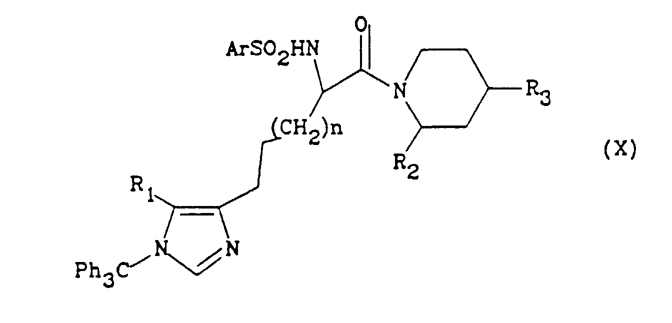 welche man einer Reaktion zur Abspaltung der Schutzgruppe unterwirft zur Bildung einer Verbindung der Formel (I bis).

6. Verfahren zur Herstellung der Verbindungen nach Anspruch 1 der Formel (I ter) 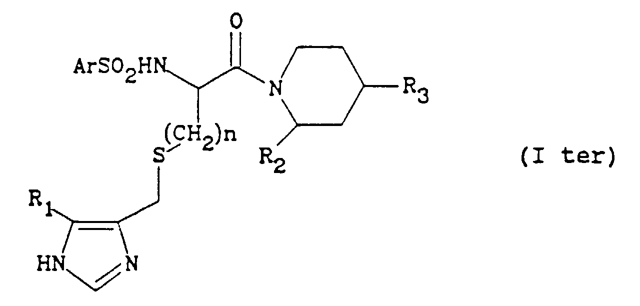 in der n = 1 bedeutet und R₁, R₂, R₃ und Ar die in Anspruch 1 angegebenen Bedeutungen besitzen, dadurch gekennzeichnet, daß man einen Alkohol der Formel (II) 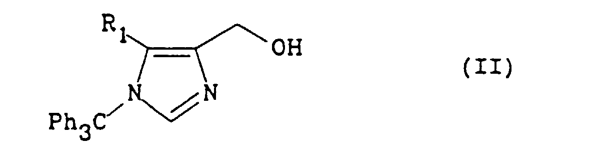 in der R₁ die in Anspruch 1 angegebenen Bedeutungen besitzt, mit Thionylchlorid umsetzt,
dann die erhaltene Verbindung mit L-Cystein umsetzt zur Bildung einer Verbindung der Formel (XII) 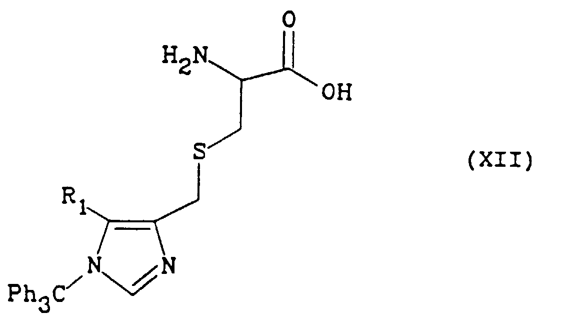 welche man mit einem Arylsulfonylchlorid der Formel
ArSO₂Cl
in der Ar die in Anspruch 1 angegebenen Bedeutungen besitzt, umsetzt zur Bildung einer Verbindung der Formel (XIII) 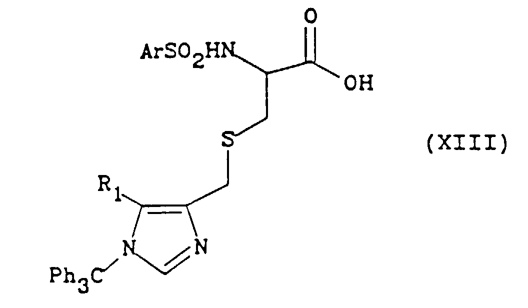 welche man mit einer Verbindung der Formel (IX) 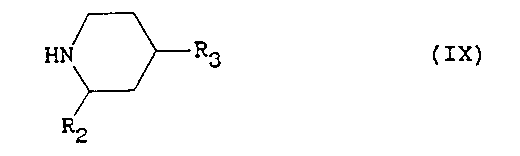 in der R₂ und R₃ die in Anspruch 1 angegebenen Bedeutungen besitzen, kondensiert,
zur Bildung einer Verbindung der Formel (XIV) 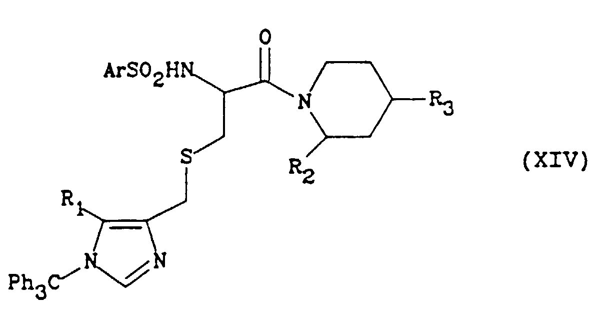 von welcher man die Schutzgruppe abspaltet zur Bildung einer Verbindung der Formel (I ter), in der n = 1 bedeutet.

7. Verfahren zur Herstellung der Verbindungen nach Anspruch 1 der Formel (I ter) 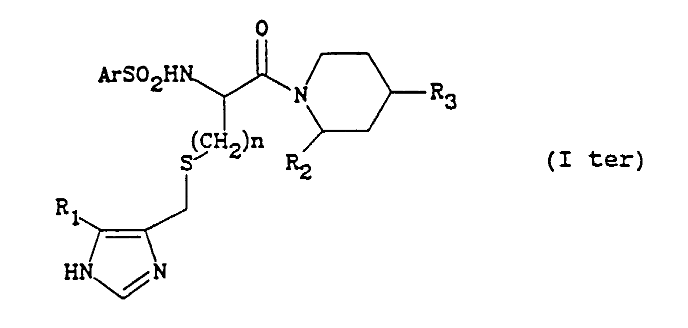 in der n = 2 bedeutet und R₁, R₂, R₃ und Ar die in Anspruch 1 angegebenen Bedeutungen besitzen, dadurch gekennzeichnet. daß man einen Alkohol der Formel (II) 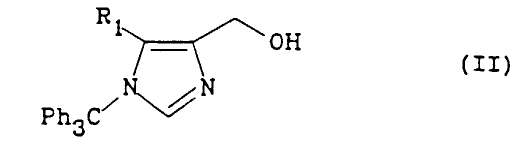 in der R₁ die in Anspruch 1 angegebenen Bedeutungen besitzt, mit Thionylchlorid umsetzt,
dann die erhaltene Verbindung mit Kaliumthioacetat umsetzt zur Bildung einer Verbindung der Formel (XV) 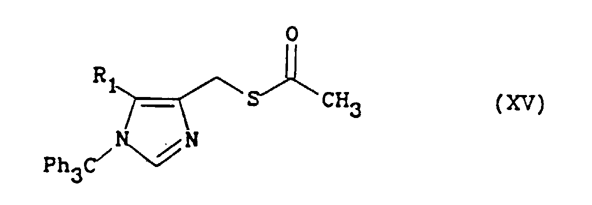 welche man mit einem Tosylat der Formel (XVI) 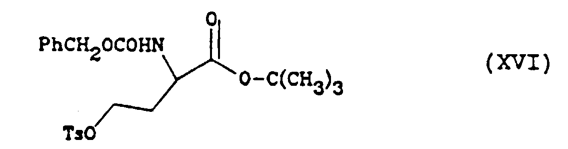 umsetzt zur Bildung einer Verbindung der Formel (XVII) 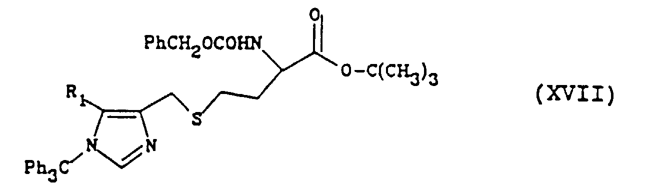 von welcher man die Schutzgruppe abspaltet zur Bildung einer Verbindung der Formel (XVIII) 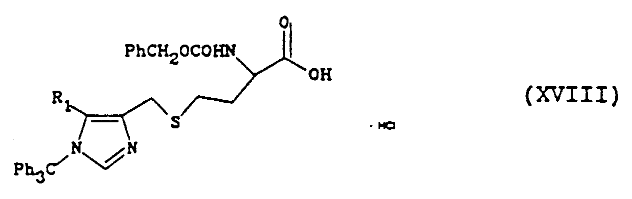 welche man mit einer Verbindung der Formel (IX) 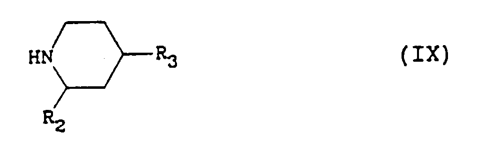 in der R₂ und R₃ die in Anspruch 1 angegebenen Bedeutungen besitzen, kondensiert,
zur Bildung einer Verbindung der Formel (XIX) 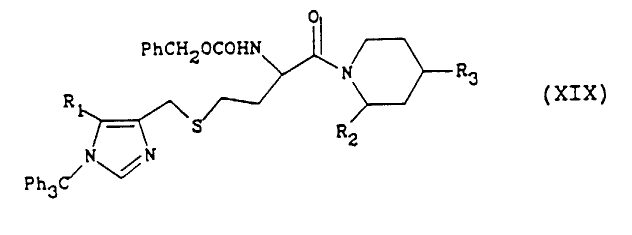 von welcher man die Schutzgruppe abspaltet zur Bildung einer Verbindung der Formel (XX) 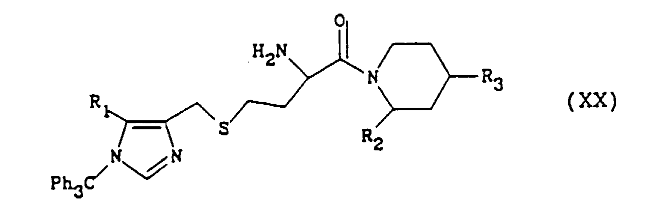 welche man mit einem Arylsulfonylchlorid der Formel
ArSO₂Cl
in der Ar die in Anspruch 1 angegebenen Bedeutungen besitzt, umsetzt zur Bildung einer Verbindung der Formel (XXI) 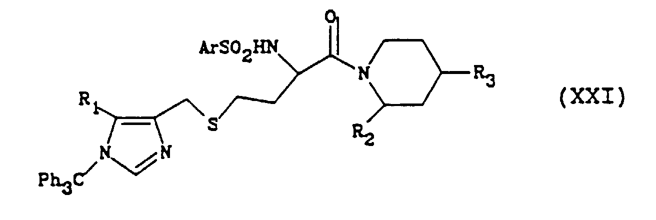 von welcher man die Schutzgruppe abspaltet zur Bildung einer Verbindung der Formel (I ter), in der n = 2 bedeutet.

8. Verfahren zur Herstellung der Verbindungen nach Anspruch 1 der Formel (I quater) 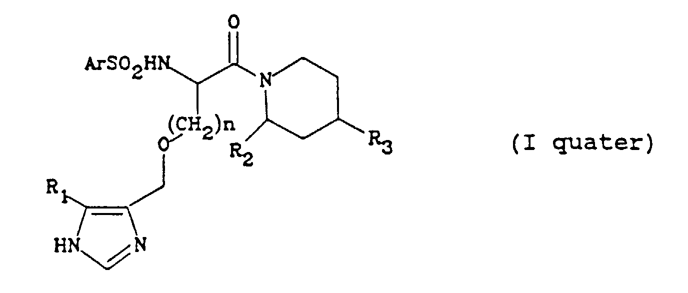 in der n = 1 oder 2 bedeutet und R₁, R₂, R₃ und Ar die in Anspruch 1 angegebenen Bedeutungen besitzen, dadurch gekennzeichnet, daß man einen Alkohol der Formel (II) 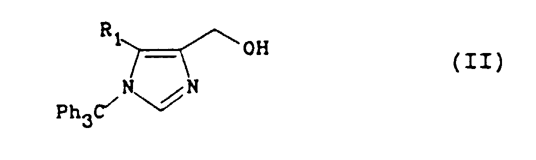 in der R₁ die in Anspruch 1 angegebenen Bedeutungen besitzt,
mit Thionylchlorid umsetzt,
dann die erhaltene Verbindung mit einer zuvor mit Natriumhydrid behandelten Verbindung der Formel (XXII) 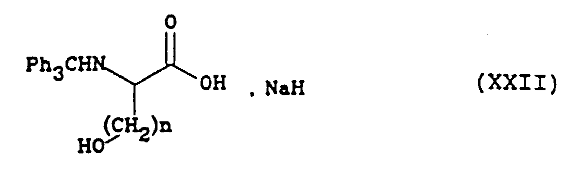 umsetzt zur Bildung einer Verbindung der Formel (XXIII) 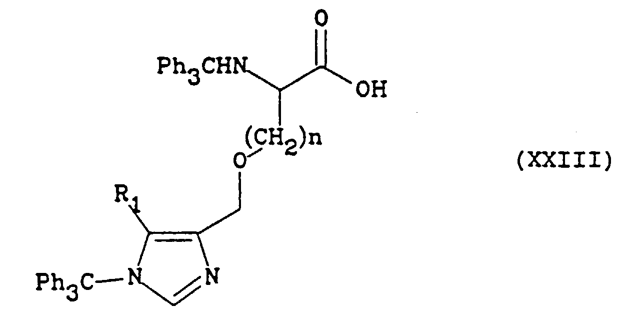 welche man mit einer Verbindung der Formel (IX) 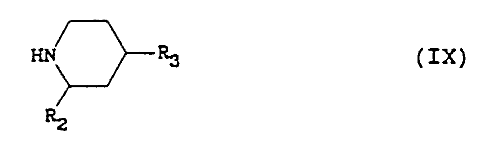 in der R₂ und R₃ die in Anspruch 1 angegebenen Bedeutungen besitzen, kondensiert
zur Bildung einer Verbindung der Formel (XXIV) 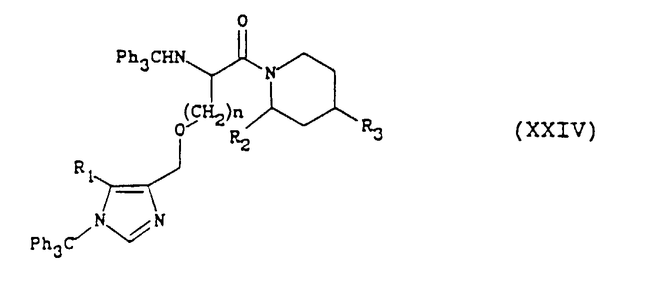 von welcher man die Schutzgruppe abspaltet zur Bildung einer Verbindung, welche man mit einem Arylsulfonylchlorid der Formel
ArSO₂Cl
in der Ar die in Anspruch 1 angegebenen Bedeutungen besitzt, umsetzt und schließlich die Schutzgruppe vom Imidazolkern abspaltet zur Bildung einer Verbindung der Formel (I quater).

9. Arzneimittel, dadurch gekennzeichnet, daß es eine Verbindung nach irgendeinem der Ansprüche 1 bis 4 enthält.

10. Pharmazeutische Zubereitung, dadurch gekennzeichnet, daß sie eine Verbindung nach einem der Ansprüche 1 bis 4 in Kombination mit irgendeinem geeigneten Trägermaterial enthält.

## Claims

1. Compounds corresponding to the formula (I) 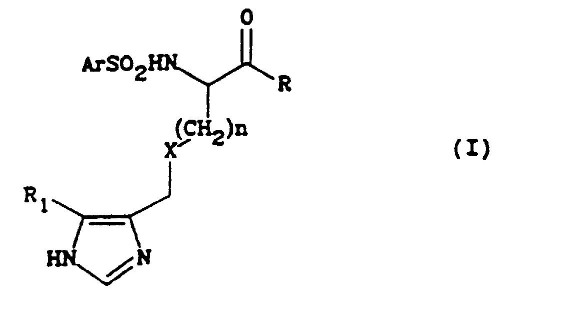 in which
R represents a group 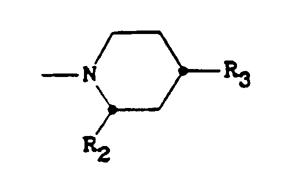 where R₂ is a hydrogen atom, a (C₁-C₄)alkoxycarbonyl group, a carboxyl group, a sodium carboxylate group, a -CH₂OR₄ group, R₄ being a hydrogen atom, a (C₁-C₄)alkyl group or a (C₁-C₄)acyl group, an amide group of formula -CONR₅R₆ or a -CN₄R₅ group, R₅ being a hydrogen atom or a (C₁-C₄)alkyl group and R₆ being a hydrogen atom, a (C₁-C₄)alkyl group, a hydroxyl group, a (C₁-C₄)alkoxy group or a (C₁-C₃)alkoxyphenyl group, and R₃ is a (C₁-C₄)alkyl group,
R₁ represents either a hydrogen atom or a (C₁-C₄)alkyl group,
X represents either a sulphur atom or an oxygen atom or a methylene group,
n = 1 or 2 and
Ar represents either a naphth-1-yl group substituted by a di(C₁-C₄)alkylamino group, or a 6,7-di(C₁-C₄)alkoxynaphth-1-yl group or a quinol-8-yl group substituted at the 3 position by a (C₁-C₄)alkyl group or a 1,2,3,4-tetrahydroquinol-8-yl group substituted at the 3 position by a (C₁-C₄)alkyl group or a 1*H*-indazol-7-yl group
and their pharmaceutically acceptable organic or inorganic salts.

2. Compounds according to Claim 1, characterized in that R represents a group 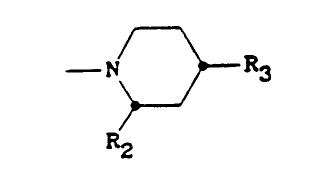 where
R₂ is a hydrogen atom, a (C₁-C₄)alkoxycarbonyl group, a carboxyl group, a sodium carboxylate group, a -CH₂OR₄ group, R₄ being a hydrogen atom, a (C₁-C₄)alkyl group or a (C₁-C₄)acyl group, an amide group of formula -CONR₅R₆ or a -CN₄R₅ group, R₅ being a hydrogen atom or a (C₁-C₄)alkyl group and R₆ being a hydrogen atom, a (C₁-C₄)alkyl group, a hydroxyl group, a (C₁-C₄)alkoxy group or a (C₁-C₃)alkoxyphenyl group, and R₃ is a (C₁-C₄)alkyl group,
R₁ represents either a hydrogen atom or a (C₁-C₄)alkyl group,
X represents either a sulphur atom or an oxygen atom or a methylene group,
n = 1 or 2 and
Ar represents either the 5-(dimethylamino)naphth-1-yl group or the 6,7-dimethoxynaphth-l-yl group or the 3-methylquinol-8-yl group or the 3-methyl-1,2,3,4-tetrahydroquinol-8-yl group
and which can optionally exist in the form of diastereoisomers.

3. Compounds according to Claim 2, characterized in that R represents a group 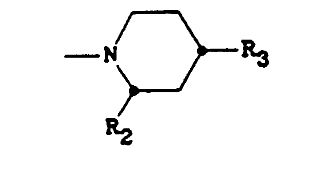 where
R₂ is a carboxyl group, an ethoxycarbonyl group, a hydroxycarboxamide group, a hydroxymethyl group or a 1*H*-tetrazolyl group and R₃ is a methyl group or an ethyl group,
R₁ is a hydrogen atom or a methyl group,
X is a methylene group n = 1 and
Ar is the 3-methyl-1,2,3,4-tetrahydroquinol-8-yl radical.

4. Compounds according to any one of Claims 1 to 3, characterized in that the configuration of the piperidyl group is [2R, 4R] and the configuration of the asymmetric carbon of the central amine-containing part 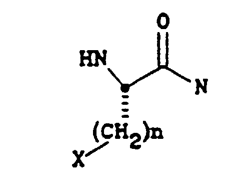 is [S].

5. Process for the preparation of the compounds according to Claim 1 of general formula (Ia) 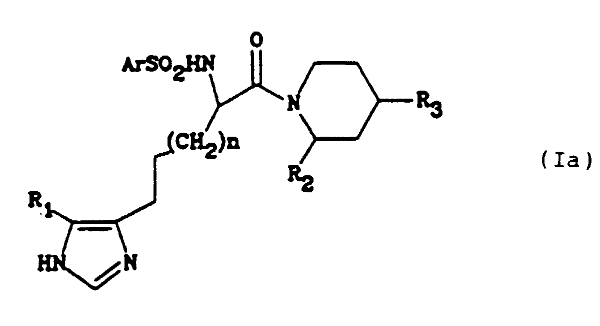 in which n = 1 or 2 and R₁, R₂, R₃ and Ar are as defined in Claim 1, which process is characterized in that an alcohol of formula (II) 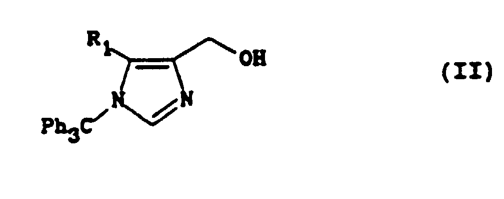 in which R₁ is as defined in Claim 1,
is reacted with thionyl chloride,
the compound obtained is then reacted with triphenylphosphine in order to obtain a compound of formula (III) 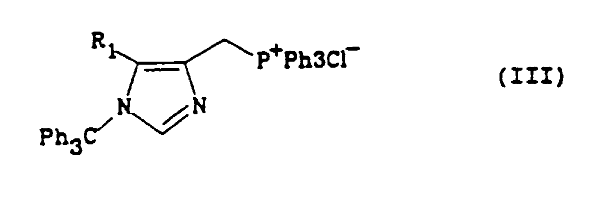 which is reacted with a compound of formula (IV) 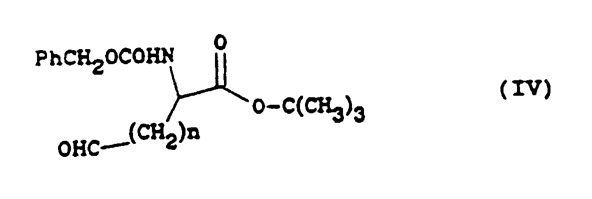 in order to obtain a compound of formula (V) 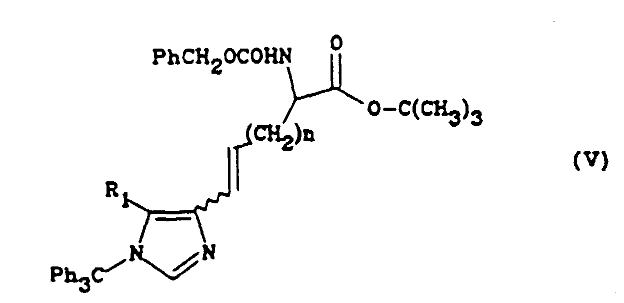 which is subjected to catalytic hydrogenation in order to obtain a compound of formula (VI) 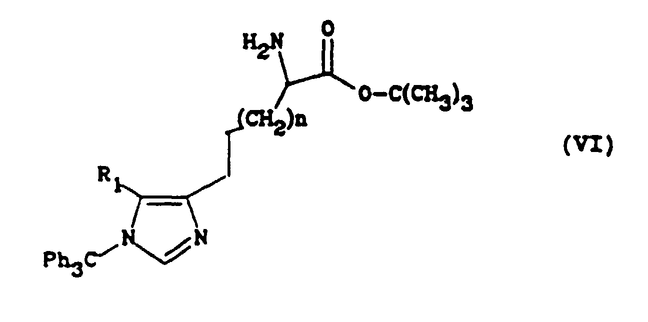 which is reacted with an arylsulphonyl chloride of formula
ArSO₂Cl
in which Ar is as defined in Claim 1,
in order to obtain a compound of formula (VII) 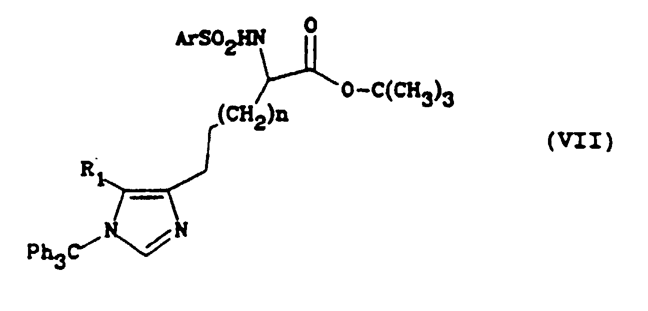 which is deprotected in order to obtain a compound of formula (VIII) 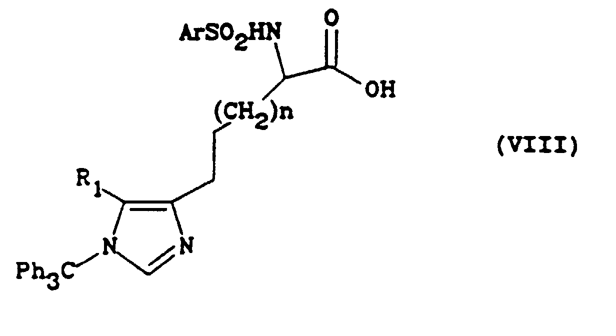 which is condensed with a compound of formula (IX) 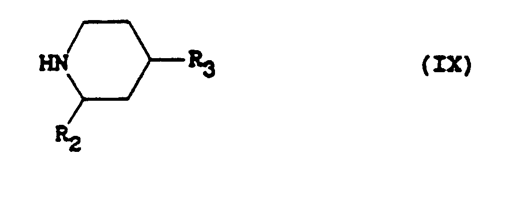 in which R₂ and R₃ are as defined in Claim 1,
in order to obtain a compound of formula (X) 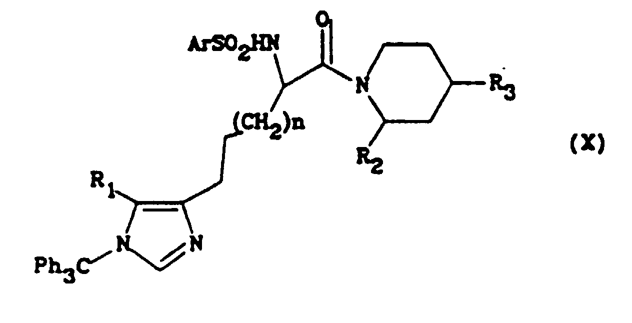 which is subjected to a deprotection reaction in order to obtain a compound of formula (Ia).

6. Process for the preparation of the compounds according to Claim 1 of formula (Ib) 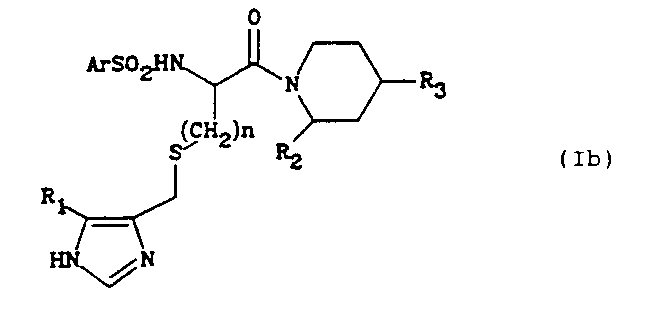 in which n = 1 and R₁, R₂, R₃ and Ar are as defined in Claim 1, which process is characterized in that an alcohol of formula (II) 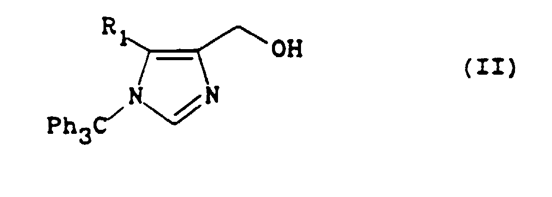 in which R₁ is as defined in Claim 1,
is reacted with thionyl chloride,
the compound obtained is then reacted with *L*-cysteine in order to obtain a compound of formula (XII) 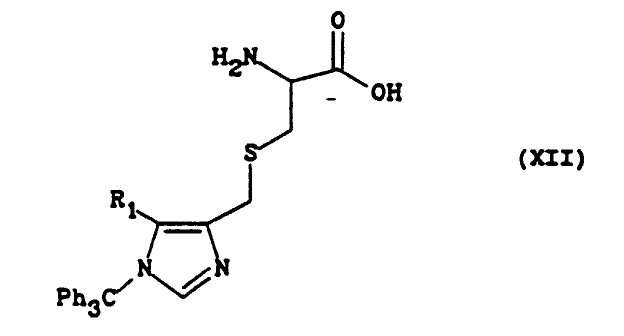 which is reacted with an arylsulphonyl chloride of formula
ArSO₂Cl
in which Ar is as defined in Claim 1,
in order to obtain a compound of formula (XIII) 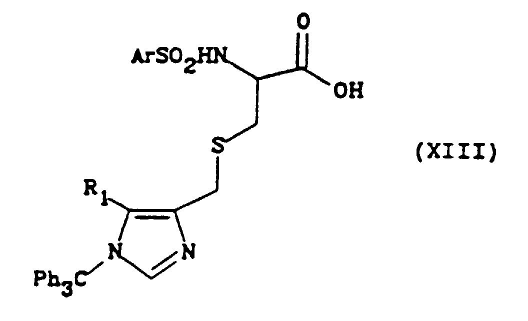 which is condensed with a compound of formula (IX) 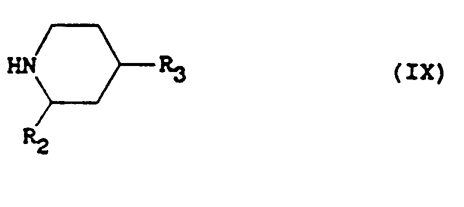 in which R₂ and R₃ are as defined in Claim 1,
in order to obtain a compound of formula (XIV) 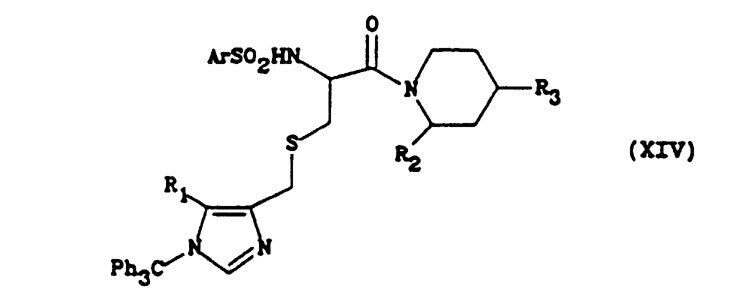 which is deprotected in order to obtain a compound of formula (Ib) in which n = 1.

7. Process for the preparation of the compounds according to Claim 1 of formula (Ib) 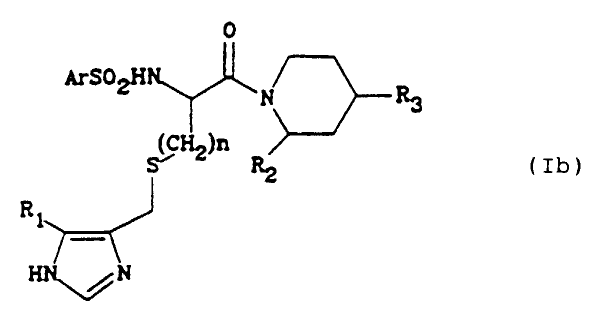 in which n = 2 and R₁, R₂, R₃ and Ar are as defined in Claim 1, which process is characterized in that an alcohol of formula (II) 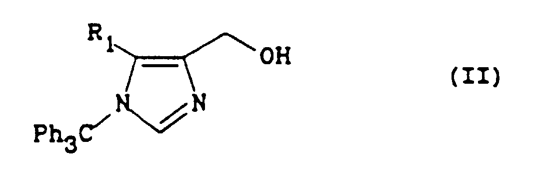 in which R₁ is as defined in Claim 1,
is reacted with thionyl chloride,
the compound obtained is then reacted with potassium thioacetate in order to obtain a compound of formula (XV) 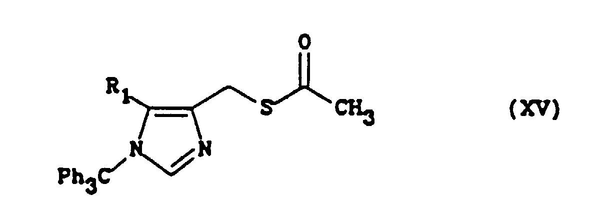 which is reacted with a tosylate of formula (XVI) 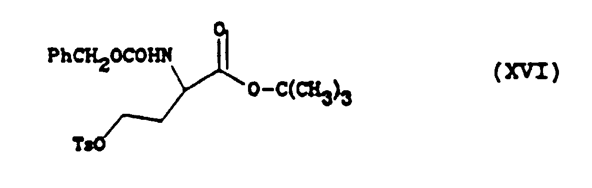 in order to obtain a compound of formula (XVII) 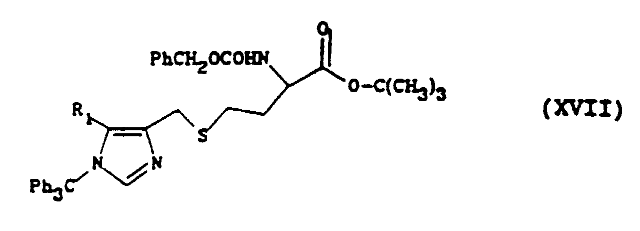 which is deprotected in order to obtain a compound of formula (XVIII) 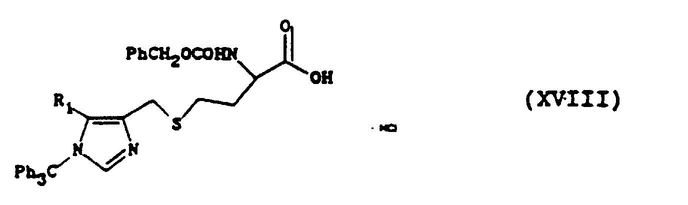 which is condensed with a compound of formula (IX) 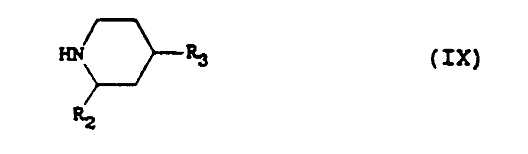 in which R₂ and R₃ are as defined in Claim 1,
in order to obtain a compound of formula (XIX) 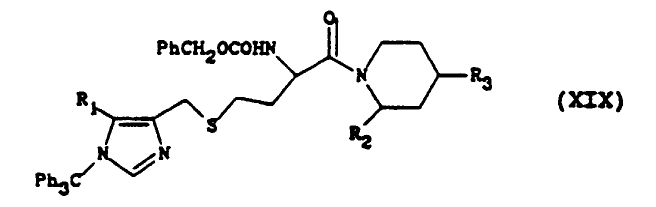 which is deprotected in order to obtain a compound of formula (XX) 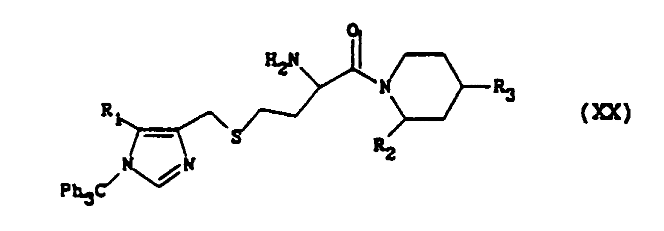 which is reacted with an arylsulphonyl chloride of formula
ArSO₂Cl
in which Ar is as defined in Claim 1,
in order to obtain a compound of formula (XXI) 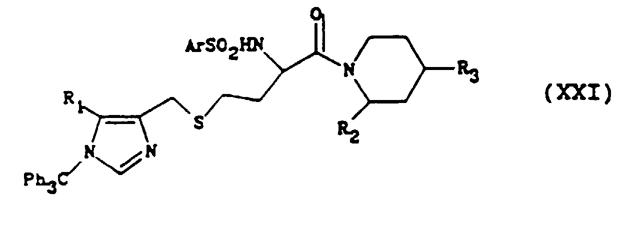 which is deprotected in order to obtain a compound of formula (Ib) in which n = 2.

8. Process for the preparation of the compounds according to Claim 1 of formula (Ic) 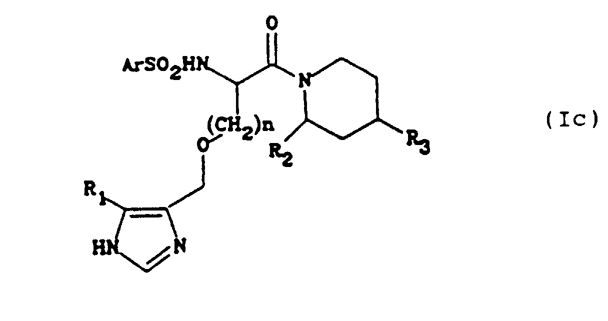 in which n = 1 or 2 and R₁, R₂, R₃ and Ar are as defined in Claim 1, which process is characterized in that an alcohol of formula (II) 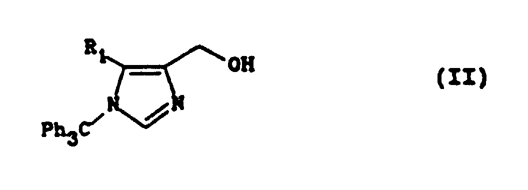 in which R₁ is as defined in Claim 1,
is reacted with thionyl chloride,
the compound obtained is then reacted with a compound of formula (XXII) which has been pretreated with sodium hydride 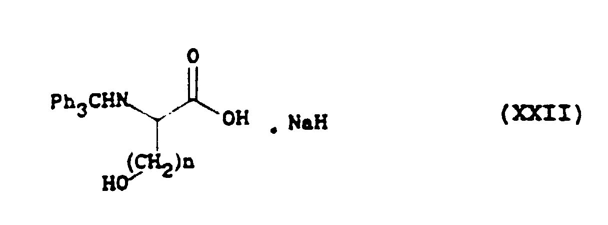 in order to obtain a compound of formula (XXIII) 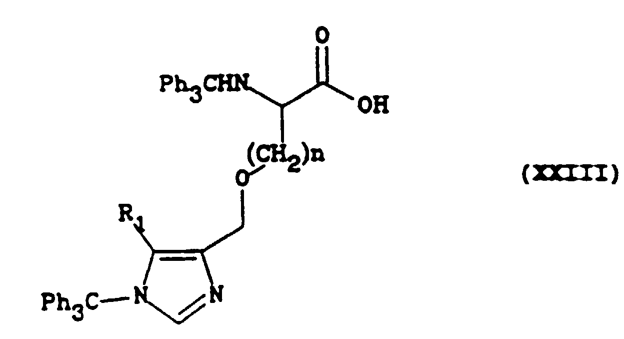 which is condensed with a compound of formula (IX) 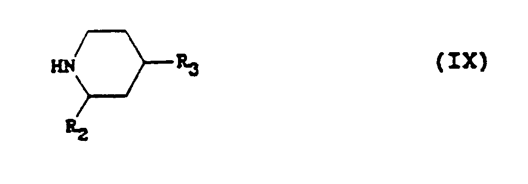 in which R₂ and R₃ are as defined in Claim 1,
in order to obtain a compound of formula (XXIV) 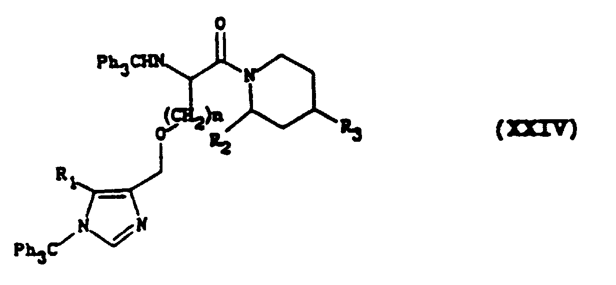 which is deprotected in order to obtain a compound which is reacted with an arylsulphonyl chloride of formula
ArSO₂Cl
in which Ar is as defined in Claim 1,
and, finally, the imidazole ring is deprotected in order to obtain a compound of formula (Ic).

9. Medicament, characterized in that it contains a compound according to any one of Claims 1 to 4.

10. Pharmaceutical composition, characterized in that it contains a compound according to any one of Claims 1 to 4 in combination with any appropriate excipient.
